(19) Europäisches Patentamt / European Patent Office / Office européen des brevets

(11) **EP 2 550 961 A1**

(12) **EUROPEAN PATENT APPLICATION**
published in accordance with Art. 153(4) EPC

(43) Date of publication:
**30.01.2013 Bulletin 2013/05**

(51) Int Cl.:
**A61K 9/46** (2006.01)     **A61K 9/28** (2006.01)
**A61K 9/70** (2006.01)     **A61K 47/02** (2006.01)
**A61K 47/04** (2006.01)     **A61K 47/30** (2006.01)
**A61K 47/38** (2006.01)

(21) Application number: **11759259.2**

(22) Date of filing: **15.03.2011**

(86) International application number:
**PCT/JP2011/056055**

(87) International publication number:
**WO 2011/118453 (29.09.2011 Gazette 2011/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2010  JP 2010066649**
**23.03.2010  JP 2010066648**

(71) Applicants:
• **Lintec Corporation**
**Tokyo 173-0001 (JP)**

• **ASKA Pharmaceutical Co., Ltd.**
**Minato-ku, Tokyo 108-8532 (JP)**

(72) Inventor: **TAKANO Youichi**
**Tokyo 173-0001 (JP)**

(74) Representative: **von Kreisler Selting Werner**
**Deichmannhaus am Dom**
**Bahnhofsvorplatz 1**
**50667 Köln (DE)**

(54) **SOLID PREPARATION**

(57)     The present invention relates to a solid preparation having an easily controllable elution property of a drug, and a method for improving dissolution of a drug.

A solid preparation 1 comprises a drug-containing unit 2 containing a drug, and a gel-forming layer 4 for covering the drug-containing unit 2 and forming a gel by water absorption, and optionally an intermediate layer 3 interposed between the drug-containing unit 2 and the gel-forming layer 4. The solid preparation 1 improves the elution property of the drug by incorporating an effervescent agent (e.g., sodium hydrogencarbonate) into the drug-containing unit 2 and/or the intermediate layer 3. The drug-containing unit 2 may contain a cationic or basic drug. The gel-forming layer 4 may contain an anionic or acidic polymer. The gel-forming layer 4 may be covered with a surface layer (anti-adhesive layer) 5.

Fig. 1

**Description**

TECHNICAL FIELD

The present invention relates to a solid preparation (particularly, a solid preparation suitable for oral administration) and a method for improving dissolution (or dissolution rate) of a drug from the solid preparation.

BACKGROUND ART

As an oral administration preparation, for example, a solid preparation in the form of a solid and a semisolid preparation in the form of a jelly (or a gel) are known. As an example of the solid preparation, an effervescent tablet is known. For example, Japanese Patent Application Laid-OpenPublicationNo. 63-264518 (JP-63-264518A, Patent Document 1) discloses an effervescent tablet comprising a carbonate or a bicarbonate, an organic acid (such as citric acid, tartaric acid, or succinic acid), and dextrin or α-starch as essential components. Moreover, Japanese Patent Application Laid-Open Publication No. 7-277959 (JP-7-277959A, Patent Document 2) discloses an effervescent tablet containing a carbonate and an acidic substance, wherein the tablet comprises not less than 6% by weight of sodium metaphosphate.

However, it is difficult for the tablet containing a carbonate and an acidic substance to achieve both compression moldability and storage stability, and the preparation design is largely limited. Moreover, these tablets are unsuitable for oral administration due to foaming immediately after contacting with water. The comfortability of taking these tablets is lowered. In addition, these tablets have a difficulty in controlling the elution property of a drug contained therein.

Moreover, an enteric-coated effervescent tablet is also known. For example, Japanese Patent No. 4372347 (JP-4372347B, Patent Document 3) discloses an enteric preparation containing glycyrrhizin or a medically acceptable salt thereof, an absorbefacient, and an effervescent agent.

However, a solid preparation such as a tablet (particularly, a bulk tablet such as a coated tablet) is usually difficult to swallow as it is, and it is necessary to take the solid preparation with a large quantity of water. In particular, it is often difficult for elderly people and infants to swallow the solid preparation. Moreover, the solid preparation has a risk of blocking the respiratory tract by accident or a risk of adhering to the esophagus.

Further, as another example of the solidpreparation, a film-shaped solid preparation is also known. For example, WO 2002/087622 (Patent Document 4) and WO 2005/097080 (Patent Document 5) describe a preparation (or a film-shaped preparation) comprising a water-swellable gel-forming layer which absorbs saliva and swells to form a gel, as an outermost layer which covers a drug-containing layer. Since the water-swellable gel-forming layer absorbs water to form the gel layer, this solid preparation can be taken with a small quantity of water.

However, the elution property of the drug from the solid preparation is sometimes significantly decreased probably because network gel fragments etc. remain in the neighborhood of the drug after disintegration of the solid preparation. In particular, when the water-swellable gel-forming layer is formed with an anionic or acidic polymer and the drug-containing layer contains a cationic or basic drug, the elution property (or dissolution rate) of the drug is drastically lowered.

On the other hand, the semisolid preparation is easy to swallow because of the jelly form thereof and is also easily administered to elderly people and infants. However, since the semisolid preparation contains a large quantity of water, the semisolid preparation confronts a problem that a drug contained in the preparation is easily decomposed or changed in quality.

RELATED ART DOCUMENTS

PATENT DOCUMENTS

Patent Document 1: JP-63-264518A (Claims)
Patent Document 2: JP-7-277959A (Claims)
Patent Document 3: JP-4372347B (Claims)
Patent Document 4: WO 2002/087622 (Claims)
Patent Document 5: WO 2005/097080(Claims)

## SUMMARY OF THE INVENTION

### PROBLEMS TO BE SOLVED BY THE INVENTION

It is therefore an object of the present invention to provide a solid preparation having an easily controllable (or improvable) elution property (or dissolution rate) of a drug, and a method for improving dissolution of the drug.

Another object of the present invention is to provide a solid preparation having an improvable elution property (or dissolution rate) of a drug even when an anionic or acidic polymer is contained in a water-swellable gel-forming layer and a cationic or basic drug is contained in a drug-containing layer, and a method for improving dissolution of the drug.

### MEANS TO SOLVE THE PROBLEMS

The inventors of the present invention made intensive studies to achieve the above objects and finally found that dissolution (or dissolution rate) of a drug from a solid preparation capable of absorbing water and forming a gel is easily controllable by action of an effervescent agent (or a foaming agent). The present invention was accomplished based on the above findings.

That is, a solid preparation in accordance with an aspect of the present invention comprises a drug-containing unit containing a drug, and a gel-forming layer for covering the drug-containing unit and forming a gel by water absorption. The solid preparation contains a physiologically acceptable effervescent agent (or foaming agent) in the drug-containing unit. Moreover, a solid preparation in accordance with another aspect of the present invention comprises a drug-containing unit containing a drug, a gel-forming layer for covering the drug-containing unit, and an intermediate layer interposed between the drug-containing unit and the gel-forming layer. The solid preparation contains a physiologically acceptable effervescent agent (or foaming agent) in at least one of the drug-containing unit and the intermediate layer. The effervescent agent may comprise a salt of at least one member selected from the group consisting of an alkali metal, an alkaline earth metal, and ammonia, wherein the salt is a carbonate, a hydrogen carbonate (or bicarbonate), or a sesquicarbonate. The ratio of the effervescent agent contained in the drug-containing unit may be about 0.1 to 160 parts by mass relative to 1 part by mass of the drug. The ratio of the effervescent agent contained in the intermediate layer may be about 0.01 to 50 parts by mass relative to 1 part by mass of the drug.

The drug-containing unit (the drug-containing unit and/or the intermediate layer when the solid preparation comprises the intermediate layer) may contain a disintegrant [for example, an acidic (or acidic group-containing) disintegrant]. The disintegrant may comprise at least one member selected from the group consisting of a carboxymethyl cellulose, a carboxymethyl starch, and a cellulose acetate phthalate. In the whole solid preparation, the ratio of the disintegrant may be about 10 to 80 parts by mass relative to 100 parts by mass of the effervescent agent.

The drug-containing unit (the drug-containing unit and/or the intermediate layer when the solid preparation comprises the intermediate layer) may contain a pharmaceutically acceptable electrolyte. The combination of the effervescent agent and the electrolyte can further improve the elution property of the drug. The electrolyte may comprise at least one of the group consisting of an alkali metal chloride, an alkaline earth metal chloride, an alkali metal phosphate, an alkaline earth metal phosphate, an alkali metal acetate, an alkali metal hydroxycarboxylate (e.g., an alkali metal lactate), an alkaline earth metal acetate, and an alkaline earth metal hydroxycarboxylate (e.g., an alkaline earth metal lactate). In particular, the electrolyte may be a phosphate such as an alkaline earth metal phosphate.

According to the solid preparation of the present invention, the drug-containing unit may contain a cationic or basic drug (hereinafter, the cationic or basic drug may be referred to as a cationic drug generically), and the gel-forming layer may contain an anionic or acidic polymer (hereinafter, the anionic or acidic polymer may be referred to as an anionic polymer generically). Even for the pharmaceutical preparation, the effervescence of the effervescent agent allows a gel, which is formed round the drug-containing unit from the gel-forming layer by absorbing water, to be disintegrated rapidly, and the elution property of the drug is improvable.

Further, the solid preparation may further comprise a surface layer (an anti-adhesive layer) for covering the gel-forming layer directly or indirectly and dissolving in water to prevent adhesion of the solid preparation to an inner wall of an oral cavity. The solid preparation may be a preparation in the form of a film.

The present invention also includes a method for improving dissolution of a drug from a solid preparation which comprises a drug-containing unit containing the drug, and a gel-forming layer for covering the drug-containing unit and forming a gel by water absorption, and the method comprises incorporating a physiologically acceptable effervescent agent into the drug-containing unit. Moreover, the present invention includes a method for improving dissolution of a drug from a solid preparation which comprises a drug-containing unit containing the drug, a gel-forming layer for covering the drug-containing unit and forming a gel by water absorption, and an intermediate layer interposed between the drug-containing unit and the gel-forming layer, and the method comprises incorporating a physiologically acceptable effervescent agent into at least one of the drug-containing unit and the intermediate layer.

EFFECTS OF THE INVENTION

According to the present invention, in the solid preparation capable of absorbing water (a small quantity of water) and forming a gel (gelation-type solid preparation), when the effervescent agent is contained in the drug-containing unit, the elution property of the drug from the solid preparation (for example, the elution property of the drug from the solid preparation at an initial stage of disintegration of the solid preparation) is easily controllable (or improvable). Moreover, when the effervescent agent is contained in the intermediate layer interposed between the drug-containing unit and the gel-forming layer, the elution property of the drug is controllable (or improvable) while inhibiting the effect of the effervescent agent on the drug. In particular, even if the solid preparation comprises the water-swellable gel-forming layer containing the anionic polymer and the drug-containing unit (or layer) containing the cationic drug, the effervescence of the effervescent agent allows a gel, which is formed round the drug-containing unit from the gel-forming layer by water absorption, to be disintegrated rapidly, and the elution property of the drug is improvable. Further, when both the effervescent agent and the electrolyte are contained in the solid preparation of the present invention, the elution property of the drug is further improvable. The solid preparation allows unpleasant taste and smell of the drug to be masked effectively, and the comfortability of taking the solid preparation can be drastically improved. In addition, the rapid elution after disintegration of the solid preparation allows the absorbability of the drug to be improved.

BRIEF DESCRIPTION OF DRAWINGS

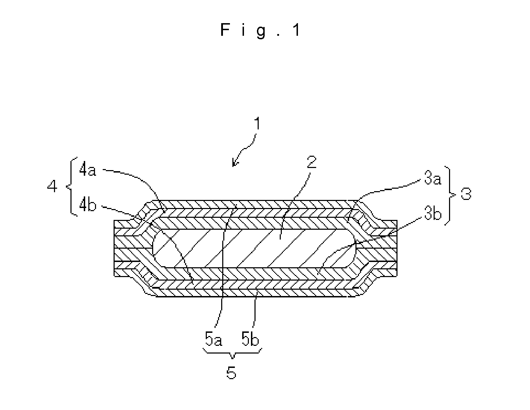

[Fig. 1] Fig. 1 is a schematic cross-sectional view showing a solid preparation in accordance with an embodiment of the present invention.

DESCRIPTION OF EMBODIMENTS

Hereinafter, the present invention will be explained in detail with reference to the attached drawings if necessary.
The solid preparation of the present invention comprises a drug-containing unit containing a drug and a gel-forming layer for covering the drug-containing unit directly or indirectly and forming a gel by swelling due to water absorption. For example, for a solid preparation comprising a gel-forming layer for covering a drug-containing unit directly, the drug-containing unit contains a physiologically acceptable effervescent agent; and for a solid preparation comprising a gel-forming layer for covering a drug-containing unit indirectly (through an intermediate layer interposed between the drug-containing unit and the gel-forming layer), the drug-containing unit and/or the intermediate layer contains a physiologically acceptable effervescent agent.
Fig. 1 shows a schematic cross-sectional view of a solid preparation in accordance with an embodiment of the present invention.
A solid preparation (an oral administration preparation) 1 shown in Fig. 1 comprises a drug-containing unit (or drug-containing layer) 2 containing a drug, an intermediate layer (or adhesive layer) 3 for covering the drug-containing unit, a gel-forming layer 4 for covering the intermediate layer and swelling by water absorption to form a gel, and a water-soluble anti-adhesive layer (or outermost layer) 5 for covering the gel-forming layer and preventing adhesion inside the oral cavity (or buccal cavity). In this embodiment, the above-mentioned layers are laminated to form a laminate. That is, the intermediate layer 3 comprises a first intermediate layer 3a laminated on a first surface of the drug-containing unit 2 and a second intermediate layer 3b laminated on a second surface of the drug-containing unit 2. The first intermediate layer 3a and the second intermediate layer 3b are adhered (or bonded) together at the periphery of the drug-containing unit 2 to seal the drug-containing unit 2. Further, the gel-forming layer 4 comprises a first gel-forming layer 4a laminated on the first intermediate layer 3a and a second gel-forming layer 4b laminated on the second intermediate layer 3b. The anti-adhesive layer 5 comprises a first anti-adhesive layer 5a laminated on the first gel-forming layer 4a and a second anti-adhesive layer 5b laminated on the second gel-forming layer 4b. In this embodiment, the drug-containing unit 2 contains an effervescent agent.
According to the solid preparation 1, the water-soluble anti-adhesive layer 5 is rapidly dissolved in water or moisture (e.g., saliva) in the oral cavity to form a lower-viscous layer (or membranous layer) on an outermost surface of the solid preparation. Thus, the gel-forming layer 4 can prevent adhesion of the solid preparation 1 to an inner wall of the oral cavity. Moreover, in the oral cavity, the gel-forming layer 4 absorbs saliva or water through the anti-adhesive layer 5 and swells to form a gel. Accordingly, even if a large quantity of water is absent, the solid preparation 1 changes into a smooth and slippery dosage form having easy-to-swallow size, shape, elasticity, viscosity, and other properties in the oral cavity, so that the solid preparation 1 can easily be administered (or given) to a patient. Moreover, the solid preparation 1 reduces a risk of blocking the respiratory tract of the patient, and thus the solid preparation 1 can safely be administered even to elderly people and infants.
Since the drug-containing unit 2 contains the effervescent agent, the elution property of the drug in the drug-containing unit 2 (further, e.g., the dispersibility of the drug and the absorbability of the drug) can significantly be improved.

Specifically, the drug-containing unit of the solid preparation 1 is covered with a plurality of layers including the gel-forming layer; when the solid preparation 1 is subjected to a dissolution test, there is a tendency to decrease the elution property of the drug. This is probably due to still covering of the drug-containing unit with a gel formed by water-absorbing and swelling of the gel-forming layer 4, even after enough absorption of water to the inside of the solid preparation 1. However, when the drug-containing unit 2 contains an effervescent agent, the disintegration of the solid preparation 1 induces an inflow of water into the drug-containing unit, thereby forming foam from the effervescent agent and generating an air flow. Probably because the air flow improves the dispersibility of the drug and rapidly separates the layer for covering the drug-containing unit to diffuse the layer far away from the drug-containing unit, the elution property of the drug can drastically be improved.

Incidentally, for the solid preparation, the intermediate layer (or adhesive layer) is not necessarily required. In order to seal the drug-containing unit by bonding (or adhesively attaching) the first gel-forming layer to the second gel-forming layer, it is preferable that the solid preparation have the intermediate layer (or adhesive layer). For the solid preparation having the intermediate layer, the intermediate layer may contain a physiologically acceptable effervescent agent. When the intermediate layer contains the effervescent agent, the elution property of the drug can be improved by an action mechanism similar to that of the solid preparation having the structure shown in Fig. 1, and the effect of the effervescent agent on the drug can be reduced.

Moreover, the solid preparation does not always require the anti-adhesive layer (surface layer). The anti-adhesive layer can effectively prevent the adhesion of the solid preparation to the inner wall of the oral cavity and improve the comfortability of taking the solid preparation.

[Drug-containing unit]

The active ingredient (drug) contained in the drug-containing unit is not particularly limited to a specific one as far as the active ingredient can be orally administered, and, for example, may be either a pharmacologically active ingredient or a physiologically active ingredient, and the pharmacologically active ingredient and the physiologically active ingredient may be used in combination. These ingredients may be solid or semisolid, and as far as the drug-containing unit maintains solid or semisolid forms thereof, a liquid active ingredient may also be used in combination. Moreover, these ingredients may be an anionic (or acidic) ingredient or a neutral ingredient or may be a cationic (or basic) ingredient.

The anionic or acidic drug contained in the drug-containing unit (hereinafter, the anionic or acidic drug may be referred to as an anionic drug generically) has at least one acidic group, such as a carboxyl group, a sulfonic acid group, or a phosphoric acid group. It is sufficient that the anionic drug has at least one acidic group. The anionic drug may have a plurality of acidic groups, which may be the same or different in species. Moreover, the drug may form a salt [for example, a salt with an alkali metal (such as sodium or potassium)]. Incidentally, the anionic or acidic drug may also include a drug which changes to be anionic or acidic by metabolism, and an anionic or acidic pro-drug which expresses an activity in a living body.

The cationic drug contained in the drug-containing unit has at least one basic group, for example, a primary amino group ($-NH_2$), a secondary amino group (imino group -NH-), a tertiary amino group (>N-), an amide group, a basic nitrogen-containing heterocyclic group (e.g., a pyrrolyl group, an imidazolyl group, a pyrazolyl group, a pyrazinyl group, a purinyl group, a quinolyl group, a pyridyl group, a piperidino group, a piperidyl group, a piperazinyl group, and a triazolo group). Incidentally, the amino group also includes a hydrazino group ($-NH-NH_2$), a hydrazo group (-NH-NH-), and others. It is sufficient that the cationic drug has at least one basic group. The cationic drug may have a plurality of basic groups, which may be the same or different in species. Moreover, the drug may form a salt [for example, a salt with an inorganic acid (e.g., hydrochloric acid, sulfuric acid, and phosphoric acid), an organic carboxylic acid (e.g., acetic acid, tartaric acid, citric acid, fumaric acid, and maleic acid), or an organic sulfonic acid (e.g., mesylic acid)]. Incidentally, the cationic or basic drug may also include a drug which changes to be cationic or basic by metabolism, and a cationic or basic pro-drug which expresses an activity in a living body.

The neutral drug contained in the drug-containing unit may include, for example, a drug free from the acidic group and the basic group. Moreover, the drug may form a salt [for example, a salt with an alkali metal (such as sodium or potassium)]. Incidentally, the neutral drug may also include a drug which neutralizes due to metabolism, and a neutral pro-drug which expresses an activity in a living body.

There are no particular limitation on the species of the pharmacologically active ingredient, and the pharmacologically active ingredient may for example be a drug which acts to a central nervous system, an autonomic nervous system, a respiratory system, a circulatory system, a digestive system, a metabolic system, or other systems; or may be a drug affecting blood and hemopoiesis, a drug used in the ophthalmologic field or the otological field, an in vivo active substance (autacoid), and others. The species of the pharmacologically active ingredient may specifically be an antipyretic (or a febrifuge), an analgesic, an antiphlogistic (or an anti inflammatory agent), a hypnotic and a sedative, a rheumatism-treating agent (or an antirheumatic), an antidepressant, an antiepileptic, an antivertigo agent, an antiallergic agent, a cardiant, a β-blocking agent, a calcium antagonist, an antiarrhythmic agent, a diuretic, an angina-treating agent, an agent for treating heart failure, an agent for treating myocardial infarction, a depressor (a hypertension-treating agent), an agent for treating disturbances of peripheral circulation, a vasopressor (a hypotension-treating agent), a bronchodilator, an antasthmatic, an antituberculousagent, a diabetic agent, an agent for treating diabetic complication, a hyperlipemia-treating agent (or a cholesterol-lowering agent), a hyperlithuria-treating agent, an antitussive expectorant, an agent for treating peptic ulcer, an agent for treating thyroid disease, a prostatomegaly-treating agent, a carcinostatic (or an anticancer agent), an osteoporosis-treating agent, an agent for treating Alzheimer's disease, an antibiotic, a vitamin compound, and an antiplasmin agent.

Concrete examples of the anionic drug as a pharmacologically active ingredient may include an antipyretic, analgesic or antiphlogistic (such as aspirin, ibuprofen, ketoprofen, sulindac, loxoprofensodiumhydrate, or zaltoprofen), an antiallergic agent (such as clomoglicic acid or seratrodast), an antiepileptic (such as valproic acid), a diuretic (such as ethacrynic acid), a hyperlipemia-treating agent [e.g., a statin compound (such as pravastatin, simvastatin, fluvastatin, atorvastatin, pitavastatin, or rosuvastatin), a fibrate compound (such as clofibrate, simfibrate, clinofibrate, bezafibrate, or fenofibrate), and dextran sulfate sodium sulfur), an agent for treating peptic ulcer (such as sofalcone, ornoprostil, sucralfate hydrate, or egualen sodium hydrate), a cathartic (or a purgative) (such as sennoside), an agent for treating hepatic disease (such as glucuronic acid), a drug acting on the autonomic nervous system (such as trepibutone), an agent for treating anemia (such as ferrous sulfate or sodium ferrous citrate), a hormone and an endocrine-therapeutic agent (such as prednisolone), an antibiotic (such as fosfomycin), and a vitamin compound (such as tocopherol succinate), or a pharmaceutically acceptable salt thereof.

Concrete examples of the cationic drug as a pharmacologically active ingredient may include an antipyretic, analgesic or antiphlogistic [e.g., an antipyretic analgesic (such as dimetotiazine mesilate), an anticephalalgic agent (such as dihydroergotamine mesilate, lomerizine hydrochloride, or sumatriptan succinate), and an antiphlogistic (such as fenamic acid, mefenamic acid, floctafenine, proglumetacin maleate, epirizole, or tiaramide hydrochloride)], an antirheumatic (such as penicillamine or methotrexate), a hyperlithuria-treating agent (such as allopurinol), a hypnotic and a sedative (such as rilmazafone hydrochloride or zolpidem tartrate), an antidepressant (such as nortriptyline hydrochloride, imipramine hydrochloride, amitriptyline hydrochloride, clomipramine hydrochloride, fluvoxamine maleate, or milnacipran hydrochloride), an antivertigo agent (such as isoprenaline hydrochloride or betahistine mesilate), an antiallergic agent (e.g., an antihistaminic agent such as diphenhydramine hydrochloride, diphenylpyraline teoclate, clemastine fumarate, chlorpheniramine maleate, alimemazine tartrate, or promethazine hydrochloride; and a histamine $H_1$ antagonist (or a basic antiallergic agent) such as ketotifen fumarate, azelastine hydrochloride, or epinastine hydrochloride), a cardiant (such as denopamine or isoprenaline hydrochloride), an antianginal agent (such as nicorandil, etafenone hydrochloride, dipyridamole, trapidil, or trimetazidine hydrochloride), a β-blocking agent (such as propranolol hydrochloride, difenidol hydrochloride, bufetolol hydrochloride, bupranolol hydrochloride, bopindolol malonate, oxprenolol hydrochloride, alprenolol hydrochloride, indenolol hydrochloride, acebutolol hydrochloride, or celiprolol hydrochloride), a calcium antagonist (such as manidipine hydrochloride, benidipine hydrochloride, amlodipine besilate, verapamil hydrochloride, or diltiazem hydrochloride), an antiarrhythmic agent (such as aprindine hydrochloride, pilsicainide hydrochloride, propafenone hydrochloride, aminodarone hydrochloride, nifekalant hydrochloride, sotalol hydrochloride, or bepridil hydrochloride), a diuretic (such as hydrochlorothiazide, penflutizide, benzylhydrochlorothiazide, bumetanide, azosemido, or triamterene), a depressor (e.g., a sympathetic blocking agent such as clonidine hydrochloride, methyldopa, guanabenz acetate, guanfacine hydrochloride, reserpine, prazosin hydrochloride, bunazosin hydrochloride, terazosin hydrochloride, or doxazosin mesilate; a vasodilator such as hydralazine hydrochloride, budralazine, todralazine hydrochloride, or cadralazine; an ACE inhibitor such as enalapril maleate, delapril hydrochloride, lisinopril, or benazepril hydrochloride; and angiotensin II receptor antagonist such as candesartan cilexetil or valsartan), an agent for treating disturbances of peripheral circulation (such asinositolhexanicotinate, hepronicate, tolazoline hydrochloride, or isoxsuprine hydrochloride), a vasopressor (such as metaraminol bitartrate, methoxamine hydrochloride, midodrine hydrochloride, amezinium metilsulfate, etilefrine hydrochloride, or phenylephrine hydrochloride), a bronchodilator and antasthmatic (e.g., a $\beta_2$-adrenergic receptor agonist such as ephedrine hydrochloride, methylephedrine hydrochloride, isoprenaline hydrochloride, orciprenaline sulfate, clorprenaline hydrochloride, salbutamol hydrochloride, terbutaline hydrochloride, formoterol fumarate, tulobuterol hydrochloride, fenoterol hydrobromide, procaterol hydrochloride, or clenbuterol hydrochloride; and a xanthine derivative such as theophylline, aminophylline, choline theophylline, or proxyphylline), an antitussive (such as dimemorfan phosphate, tipepidine hibenzate, oxeladin citrate, dextromethorphan hydrobromide, pentoxyverine citrate, chloperastine, or benproperine phosphate), a diabetic agent (such as tolbutamide, acetohexamide, glibenclamide, glimepiride, buformin hydrochloride, metformin hydrochloride, pioglitazone hydrochloride, or voglibose), an expectorant (such as L-methylcysteine hydrochloride, ambroxol hydrochloride, or bromhexine hydrochloride), an agent for treating peptic ulcer (e.g., an $H_2$ receptor antagonist such as cimetidine, ranitidine hydrochloride, or famotidine; a proton pump inhibitor such as lansoprazole or omeprazole; and a muscarine receptor antagonist such as pirenzepine hydrochloride), an antibiotic (such as clarithromycin, kitasamycin, josamycin, midecamycin, rokitamycin, or azithromycin), a narcotic (such as amphetamine or meperidine), a vitamin compound [e.g., a vitamin $B_1$ compound such as thiamine hydrochloride, thiamine nitrate, dicethiamine hydrochloride, cycotiamine, benfotiamine, bisibutiamine, fursultiamine, prosultiamine, octotiamine, bisbentiamine, or thiamine disulfide; a vitamin $B_2$ compound such asriboflavin,riboflavinsodium phosphate,riboflavin butyrate, or flavin adenine dinucleotide sodium; a vitamin $B_6$ compound such as pyridoxine hydrochloride, pyridoxine acetate, or pyridoxal phosphate; a nicotinic acid compound such as nicotinic acid, or nicotinamide; a vitamin $B_{12}$ compound such as mecobalamin, cyanocobalamin, hydroxocobalamin (such as hydroxocobalamin hydrochloride or hydroxocobalamin acetate), or methylcobalamin; folic acid, a pantothenic acid compound, biotin, and vitamin P (such as hesperidin)], and an antiplasmin agent (such as ε-aminocaproic acid or tranexamic acid).

Concrete examples of the neutral drug contained in the drug-containing unit may include a cardiant (such as digitoxin or digoxin), a diuretic (such as spironolactone), an agent for treating peptic ulcer (such as teprenone), a hyperlithuria-treating agent (such as benzbromarone), a carcinostatic (or an anticancer agent) (such as etoposide), a centrally acting skeletal muscle relaxant (such as mephenesin), a hyperlipemia-treating agent (such as probucol), an antithrombotic agent (such as warfarin potassium), a hormone and an endocrine-therapeutic agent (such as betamethasone), and a vitamin compound (such as menatetrenone, retinol palmitate, alfacalcidol), or a pharmaceutically acceptable salt thereof.

These pharmacologically active ingredients may be used alone or in combination according to the purposes of prevention, treatment, and others.

As the anionic drug as a physiologically active ingredient, there may be mentioned an organic acid or a salt thereof [for example, α-lipoic acid, L-ascorbic acid, citric acid, malic acid, tartaric acid, oxalic acid, and fumaric acid, or an alkali metal salt thereof (a sodium salt, a calcium salt)], an amino acid or a salt thereof [for example, L-glutamic acid and L-aspartic acid, or an alkali metal salt thereof (e.g., a sodium salt)], and others.

As the cationic drug as a physiologically active ingredient, there may be mentioned an amino acid or a salt thereof [for example, glycine, L-lysine, L-valine, L-alanine, L-arginine, L-cystine, and L-methionine, or an alkali metal salt thereof (e.g., a sodium salt)], a peptide or a salt thereof [for example, a peptide (such as L-lysineglutamate, or a collagen and a collagen peptide thereof), coenzyme $Q_{10}$, and L-carnitine or a salt thereof (such as a fumarate or tartrate)], a glucosamine compound (such as a chitin or a chitosan), and others.

These physiologically active ingredients may be used alone or in combination. The physiologically active ingredient may be used in combination with the pharmacologically active ingredient.

Incidentally, the anionic drug, the cationic drug, and the neutral drug may be used in combination.

Dissolution of even the cationic drug (for example, a drug having at least one basic group selected from the group consisting of a primary amino group, a secondary amino group, a tertiary amino group, and a basic nitrogen-containing heterocyclic group, or a salt thereof) among these active ingredients can be drastically improved. That is, when the anionic polymer is contained in the gel-forming layer, the cationic drug is probably adsorbed (adsorbed due to ionic interaction) on or ionic-bonded to the anionic polymer. Accordingly, the elution property of the drug from the solid preparation tends to be decreased. In contrast, according to the solid preparation of the present invention, since the effervescent agent is contained in the drug-containing unit and/or the intermediate layer interposed between the drug-containing unit and the gel-forming layer, the adsorption of the cationic drug on or to the anionic polymer can effectively be prevented (or released) due to air flow or counter ions generated by foaming of the effervescent agent. Thus the elution property of the drug from the solid preparation can significantly be improved.

According to the present invention, since the drug-containing unit can be enclosed in the gel-forming layer, a physical strength can be imparted to the solid preparation even when the solid preparation contains a relatively large amount of an active ingredient, or a bulky active ingredient, which easily lowers the physical strength of the solid preparation. Thus, the present invention can be applied to both a slight or low dose (e.g., not more than 1 mg) of an active ingredient and a large or high dose (e.g., not less than 300 mg) of an active ingredient as the active ingredient. The unit dosage amount of the active ingredient may for example be about 0.01 to 1500 mg (e.g., about 0.01 to 800 mg), preferably about 0.1 to 1200 mg (e.g., about 0.1 to 500 mg), and more preferably about 1 to 1000 mg (e.g., about 1 to 300 mg) and is usually about 1 to 500 mg (e.g., about 2 to 250 mg). The active ingredient content can be selected according to the species of the active ingredient or others, and is usually, in the drug-containing unit, about 0.001 to 99.9% by mass, preferably about 0.01 to 70% by mass (e.g., about 0.01 to 50% by mass), and more preferably about 0.1 to 35% by mass.

The solid preparation of the present invention provides a comfortable feeling to take and can effectively be administered orally with a small quantity of water or substantially without water. Thus, for example, the solid preparation can suitably be used for an active ingredient having a large unit dosage amount, a bulky active ingredient, an unpalatable (such as bitter or acerbic) active ingredient, a highly water-soluble active ingredient. Among these ingredients, usually, the pharmacologically active ingredient is widely used.

The drug-containing unit may comprise the active ingredient alone, and usually contains an additive (a base material or a carrier) in addition to the active ingredient. The additive is not particularly limited to a specific one, and depending on the shape of the preparation, a conventional carrier, for example, at least one carrier selected from the group consisting of an excipient, a binder, a lubricant, and a disintegrant may be selected.

As the excipient, there may be mentioned a saccharide such as lactose, white sugar or refined sugar, maltose, glucose, sucrose, or fructose (or fruit sugar); a sugar alcohol such as mannitol, sorbitol, or xylitol; a starch such as a corn starch or a potato starch; a polysaccharide such as a crystalline cellulose (including a microcrystalline cellulose), cyclodextrin, or dextran; silicon dioxide or a silicate such as a light silicic anhydride, a synthetic aluminum silicate, magnesium silicate,magnesium aluminometasilicate,or atalc;anoxide such as titanium oxide; a phosphate such as calcium monohydrogenphosphate; and others. The binder may include a water-soluble starch or starch derivative such as a pregelatinized starch, a partially pregelatinized starch, an oxidized starch, a sodium carboxymethyl starch, a hydroxypropyl starch, or dextrin; a polysaccharide such as agar, gum acacia (or gum arabic), dextrin, sodium alginate, a tragacanth gum, a pullulan, a xanthan gum, a hyaluronic acid, a pectin, a sodium chondroitin sulfate, or a gelatin; a synthetic polymer such as a polyvinylpyrrolidone (e.g., a povidone), a vinyl acetate-vinylpyrrolidone copolymer, apoly (vinyl alcohol), a carboxyvinylpolymer, a polyacrylic acid-seriespolymer (orapolyacrylicpolymer), apolylactic acid, a poly (ethylene glycol), or a poly(vinyl acetate); a cellulose ether such as a methyl cellulose (MC), an ethyl cellulose (EC), a carboxymethyl cellulose (CMC), a carboxymethylethyl cellulose (CMEC), a hydroxypropyl cellulose (HPC), or a hydroxypropylmethyl cellulose (HPMC), and a cellulose ester such as a cellulose acetate; and others. The lubricant may include a talc, magnesium stearate, a poly(ethylene glycol) 6000, and others. The disintegrant is not particularly limited to a specific one. The disintegrant may include, for example, a cellulose derivative such as a carboxymethyl cellulose or a salt thereof (e.g., a carmellose, a carmellose sodium, a carmellose calcium, and a croscarmellose sodium), a starch derivative such as a carboxymethyl starch, and a polyvinylpyrrolidone (e.g., a povidone and a crosslinked polyvinylpyrrolidone (crospovidone)), a low-substituted hydroxypropyl cellulose, magnesium aluminometasilicate, and others. These carriers may be used alone or in combination.

The drug-containing unit may contain a polyglucosamine compound (such as a chitin or a chitosan), a protein (such as a casein or a soybean protein), an enteric base material (e.g., a cellulose derivative such as a cellulose phthalate, a cellulose acetate phthalate, a hydroxypropyl cellulose phthalate, a hydroxypropylmethyl cellulose phthalate (HPMCF), or a hydroxypropylmethyl acetate succinate, a methacrylic acid-ethyl acrylate copolymer (methacrylic acid copolymer LD), a methacrylic acid-n-butyl acrylate copolymer, and a methacrylic acid-methyl methacrylate copolymer (methacrylic acid copolymers L and S)), a gastric-soluble base material (a dimethylaminoethyl methacrylate-methacrylic acid copolymer, a dimethylaminoethyl methacrylate-methyl methacrylate copolymer, a dimethylaminoethyl methacrylate-chlorotrimethylammoniumethyl methacrylate copolymer, a dimethylaminoethyl methacrylate-chlorotrimethylammoniummethyl methacrylate copolymer, a dimethylaminoethyl methacrylate-chlorotrimethylammoniumethyl acrylate copolymer, a polyvinylacetal diethylaminoacetate), and others. Moreover, the enteric base material and/or gastric-soluble base material may be used as the binder.

Further, the drug-containing unit may contain a fat and oil. The fat and oil may include a wax (e.g., a beeswax, acarnaubawax, acacaobutter, alanolin, aparaffin, and a petrolatum), a higher (or long chain) fatty acid ester [e.g., an alkyl ester of a saturated or unsaturated fatty acid, and an ester of a fatty acid with a polyhydric alcohol (such as a poly $(C_{2-4}$alkylene glycol), glycerin, or a polyglycerin) (e.g., a glyceride)], a hardened (or hydrogenated) oil, a higher alcohol (e.g., a saturated aliphatic alcohol such as stearyl alcohol and an unsaturated aliphatic alcohol such as oleyl alcohol), a higher fatty acid (e.g., linoleic acid, linolenic acid, oleic acid, and stearic acid), a metallic soap (e.g., a metal salt of a fatty acid, such as a sodium salt of palm oil fatty acid or calcium stearate), and others.

Furthermore, for the drug-containing unit, a known additive can be used. Such an additive may include, for example, a disintegrant aid (or adjuvant), an antioxidation agent or an antioxidant, a variety of surfactants such as a nonionic surfactant, a dispersing agent, an antiseptic agent or a preservative (e.g., a paraben such as methyl paraben or butyl paraben), a fungicide or antibacterial agent (e.g., a benzoic acid compound such as sodiumbenzoate), an antistatic agent, a corrigent or a masking agent (e.g., sweetening agent), a coloring agent (e.g., a dye and a pigment such as titanium oxide or colcothar), a deodorant or a flavoring agent (or perfume) (e.g., an aromatic substance), and an algefacient. These additives may be used alone or in combination.

The ratio of the additive may for example be about 0.001 to 100 parts by mass (e.g., about 0.01 to 50 parts by mass, preferably about 0.1 to 30 parts by mass, and more preferably about 0.5 to 20 parts by mass) relative to 1 part by mass of the active ingredient.

The drug-containing unit containing the active ingredient and the additive (base material or carrier) may be shaped or formed into various shapes or dosage forms of solid preparations, for example, powdered preparations, powders, granulated preparations (e.g., granules and microfine granules), spherical or spheroidal preparations, tablets (including sublingual tablets, orally disintegrating tablets, troches, chewable tablets, and others), capsules (including hard capsules, soft capsules, and microcapsules), and layered or film-shaped (or film-covered) preparations (or sheet-shaped preparations). The shape (or form) of the drug-containing unit may for example be a spherical shape, an ellipsoidal shape, a polyhedral or prismatic shape, a layered shape, an amorphous shape, and an aggregate of particles. Incidentally, granulation or covering of the drug with the additive (base material or carrier) in the form of granules or the like can prevent the drug (cationic drug) from contacting with the component(s) of the adjacent layer and improve the stability of the drug (cationic drug) in some cases.

According to the present invention, even when the solid preparation has a large contact surface area with the inner wall of the oral cavity due to the shape of the preparation, the solid preparation can easily be swallowed without water or with a small quantity of water. Moreover, the preparation can easily be swallowed even in spite of a high drug content and a large dosage size. Thus, the drug-containing unit may be formed as a preparation that is conventionally difficult for elderly people and infants (babies and little children) to swallow [for example, a preparation having a flat region or plateau, a preparation having a flat shape, and a large-sized tablet (e.g., a tablet having a diameter of about 5 to 15 mm, preferably about 6 to 14 mm, and more preferably about 7 to 13 mm)]. Among these shapes, the drug-containing unit may have a layered or film-like shape (e.g., a polygon such as a quadrilateral, a circle, and an ellipse). The layered drug-containing unit may for example have a thickness of about 5 $\mu$m to 5 mm, preferably about 10 $\mu$m to 3 mm, and more preferably about 100 to 1000 $\mu$m (e.g., about 100 to 500 $\mu$m).

[Intermediate layer]

When the drug-containing unit contains the after-mentioned physiologically acceptable effervescent agent, an intermediate layer (or adhesive layer) is not necessarily required between the drug-containing unit and the gel-forming layer. However, when the first and second gel-forming layers are joined (or adhered) together through the intermediate layer (or adhesive layer) at the periphery of the drug-containing unit, the intermediate layer intimately joins (or adheres) these gel-forming layers to each other, effectively prevents leakage of the active ingredient from the drug-containing unit, and allows smooth administration of the preparation.

On the other hand, when the drug-containing unit does not contain the physiologically acceptable effervescent agent, incorporation of the physiologically acceptable effervescent agent into the intermediate layer can improve the elution property of the drug while inhibiting an influence on the drug.

Incorporation of the physiologically acceptable effervescent agent into both the drug-containing unit and the intermediate layer can further improve the elution property of the drug.

Incidentally, when the drug-containing unit does not contain the after-mentioned physiologically or pharmaceutically acceptable electrolyte, incorporation of the physiologically or pharmaceutically acceptable electrolyte into the intermediate layer can improve the elution property of the drug while inhibiting an influence on the drug. Moreover, incorporation of the physiologically or pharmaceutically acceptable electrolyte into both the drug-containing unit and the intermediate layer can further improve the elution property of the drug.

The base material (adhesive agent) of the intermediate layer (or adhesive layer) may include a (meth)acrylic acid-series polymer (or a (meth)acrylic polymer) [for example, a polyacrylic acid or a salt thereof (such as a carboxyvinyl polymer or a poly (sodium acrylate)) ; and an acrylic acid copolymer or a salt thereof], a vinylpyrrolidone-series polymer [a povidone, and a copolymer of vinylpyrrolidone such as a vinyl acetate-vinylpyrrolidone copolymer], a polysaccharide [for example, a polysaccharide derived from a plant (e.g., a cellulose derivative such as a CMC, a CMC sodium salt, an MC, an HPC, or an HPMC, a karaya gum, a pectin, a guar gum, a locust bean gum, a gum acacia (or gum arabic), a tragacanth gum, a carrageenan, and alginic acid or a sodium salt thereof), and a polysaccharide derived from a fungus (e.g., an acidic polysaccharide such as a pullulan, a xanthan gum, a hyaluronic acid, or a chondroitin sulfate or a sodium salt thereof)], a vinyl acetate-series polymer (e.g., a poly(vinyl acetate) and an ethylene-vinyl acetate copolymer), a (meth)acrylic acid-series polymer [e.g., a methacrylic acid-ethyl acrylate copolymer (methacrylic acid copolymer LD), a methacrylic acid-n-butyl acrylate copolymer, and a methacrylic acid-methyl methacrylate copolymer (methacrylic acid copolymers L and S)], and others. The (meth) acrylic acid-series polymer may include the same polymer as the after-mentioned gel-forming agent or anionic polymer for the anti-adhesive layer. These adhesive agents maybe used alone or in combination. Moreover, the adhesive agent may show neutral disintegration, acidic disintegration, or basic disintegration.

The adhesive may have heat (orthermal) adhesiveness (heat-sealing property). Such an adhesive having heat adhesiveness may include a (meth)acrylic acid-series polymer, a vinylpyrrolidone-series polymer, a vinyl acetate-series polymer, and others. When an adhesive having heat adhesiveness is used, the drug-containing unit can be sealed in a simple operation by interposing the drug-containing unit between a pair of film-like adhesive layers and heat-adhering (heat-bonding) the adhesive layers each other at the periphery of the drug-containing unit.

The adhesive layer may contain a plasticizer. Examples of the plasticizer may include a water-soluble plasticizer [e.g., ethylene glycol, propylene glycol, glycerin, sorbitol, sucrose, a polyoxyethylene polyoxypropylene glycol (such as pluronic or poloxamer), a polyoxyethylene sorbitan fatty acid ester (such as polysorbate 80), and a poly(ethylene glycol) (such as a poly(ethylene glycol) having a mass-average molecular weight of 300 to 6000)], a water-insoluble plasticizer (e.g., triacetin, triethyl citrate, diethyl phthalate, dioctyl adipate, and a fatty acid such as lauric acid), and others. These plasticizers may be used alone or in combination. The preferred plasticizer includes a water-soluble plasticizer, such as glycerin.

The amount of the plasticizer may be selected according to the species of the base material (adhesive agent) of the adhesive layer, and may be about 1 to 100 parts by mass, preferably about 5 to 75 parts by mass (e.g., about 10 to 50 parts by mass), and more preferably about 15 to 50 parts by mass (e.g., about 20 to 40 parts by mass) relative to 100 parts by mass of the base material.

Further, the adhesive layer may contain a base material which specifically dissolves in a desired site of the body (for example, a gastric-soluble or enteric base material as exemplified in the paragraph of the drug-containing unit). Incorporation of such a base material allows the effervescent agent to certainly form foam in a desired site of the body.

The adhesive layer may cover (or coat) at least part of the surface of the drug-containing unit to adhere (or bond) the drug-containing unit to the gel-forming layer. The adhesive layer may usually cover (or coat) the whole or part of the surface of the drug-containing unit (for example, at least upper and under surfaces of a layered drug-containing unit).

The thickness of the adhesive layer may be selected from a wide range of, for example, about 1 $\mu$m to 1 mm (e.g., about 5 to 500 $\mu$m) as far as the drug-containing unit is not exposed. The thickness of the adhesive layer may be about 10 to 500 $\mu$m (e.g., about 15 to 300 $\mu$m), preferably about 20 to 200 $\mu$m (e.g., about 30 to 175 $\mu$m), and more preferably about 50 to 150 $\mu$m.

[Gel-forming layer]

The gel-forming layer is not particularly limited to a specific one as far as the layer swells with a small quantity of water (such as saliva) to form a gel. The gel-forming layer usually contains an anionic or acidic polymer as a gel-forming agent. The gel-forming layer encloses (or wraps) the drug-containing unit and gelates by a small quantity of water such as saliva, so that the gel-forming layer changes a shape or surface characteristic of the preparation to impart a significantly improved slipperiness and an elasticity or viscosity suitable for easy swallowing to the preparation. Thus the comfortability (or feeling) of taking the preparation is improved (for example, the gel-forming layer facilitates the swallowing of the preparation).

It is sufficient that the gel-forming agent of the gel-forming layer contains at least a pharmaceutically acceptable anionic or acidic polymer which may be a synthetic polymer, a cellulose derivative, a starch derivative, a natural polysaccharide, and others. The anionic or acidic polymer for the gel-forming agent may include a carboxyl group-containing polymer (or macromolecule) [for example, a synthetic polymer such as a carboxyl group-containing polymer obtainable from at least one polymerizable monomer selected from the group consisting of (meth)acrylic acid and itaconic acid as a polymerizable component, or a carboxyvinyl polymer; a cellulose derivative such as a CMC, a carboxymethylethyl cellulose, or a carboxymethylhydroxyethyl cellulose; a starch derivative such as a carboxymethyl starch; and a natural polysaccharide such as alginic acid, a heparin, a hyaluronic acid, a pectin, a cellulose derivative (such as a tragalose), a hyaluronic acid, a carrageenan, or a chondroitin sulfate], a phosphoric acid group-containing polymer (e.g.,a cellulose derivative such as a cellulose phosphate), or a salt thereof, and others. These anionic or acidic polymers may be used alone or in combination.

The anionic polymer may form, for example, a salt with an inorganic base [an alkali metal (such as sodium or potassium), ammonia] or an organic base [such as monoethanolamine, diethanolamine, triethanolamine, or dimethylaminoethanol].

Among these anionic polymers, in order to absorb water or moisture rapidly, it is preferred to use a water-soluble anionic polymer, for example, a carboxy group-containing polymer and a sulfonic acid group-containing polymer, particularly an anionic polymer comprising a (meth)acrylic acid unit as an essential polymerizable component (a homo- or copolymer of (meth) acrylic acid, or a (meth) acrylic acid-series polymer). As a monomer copolymerized with (meth) acrylic acid (copolymerizable monomer), there may be mentioned an alkyl (meth)acrylate [for example, a $C_{1-6}$alkyl (meth) acrylate such as methyl (meth)acrylate, ethyl (meth)acrylate, or butyl (meth)acrylate, particularly a $C_{1-4}$alkyl (meth) acrylate], a hydroxyalkyl (meth)acrylate [for example, a hydroxy$C_{2-4}$alkyl (meth)acrylate such as hydroxyethyl (meth) acrylate or hydroxypropyl (meth)acrylate, particularly a hydroxy$C_{2-3}$alkyl (meth)acrylate], vinyl acetate, vinylpyrrolidone, and others. These copolymerizable monomers may be used alone or in combination.

The mass ratio of the (meth) acrylic acid relative to the copolymerizable monomer may for example be about 100/0 to 50/50, preferably about 100/0 to 60/40 (e.g., about 99.9/0.1 to 65/35), and more preferably about 100/0 to 70/30 (e.g., about 99/1 to 80/20), in a ratio of the (meth) acrylic acid/the copolymerizable monomer.

The (meth) acrylic acid-series polymer may include a poly((meth)acrylic acid), a (meth)acrylic acid-methyl (meth)acrylate copolymer, a (meth)acrylic acid-ethyl (meth)acrylate copolymer, a (meth)acrylic acid-butyl (meth)acrylate copolymer, and others. These (meth) acrylic acid-series polymers may be used alone or in combination.

Representative examples of the (meth)acrylic acid-series polymer includes a carboxyvinyl polymer (trade name: CARBOPOL), a poly(sodium acrylate), a partially neutralized product of a polyacrylic acid, a methacrylic acid-n-butyl acrylate copolymer, and a methacrylic acid copolymer LD (trade name: EUDRAGIT L-30D55). Among these (meth) acrylic acid-series polymers, a polyacrylic acid or an acrylic acid copolymer in each of which acrylic acid as a main monomer is polymerized (that is, an acrylic acid-series polymer), particularly a carboxyvinyl polymer, is preferred. As the carboxyvinyl polymer, there may be mentioned CARBOPOL 981, CARBOPOL 980, CARBOPOL 974P, CARBOPOL 971P, CARBOPOL 941, CARBOPOL 940, CARBOPOL 934P, CARBOPOL 71G (manufactured by Noveon, US), HIVISWAKO 103, HIVISWAKO 104 (manufactured by Wako Pure Chemical Industries, Ltd.), JUNLON (Nihon Junyaku Co., Ltd.), AQUPEC (Sumitomo Seika Chemicals Company Limited), and others.

The anionic polymer (e.g., a carboxyvinyl polymer) may have a viscosity of about 1500 to 50000 mPa·s, preferably about 2500 to 20000 mPa·s, more preferably about 5000 to 15000 mPa·s, and particularly about 7500 to 12500 mPa·s for a 0.2% by mass aqueous solution at 20°C.

Incidentally, if necessary, the anionic polymer may be used in combination with other gel-forming agents, for example, a protein (such as a collagen or a casein), a hydroxyl group-containing polymer (e.g., a synthetic polymer such as a poly(vinyl alcohol), a cellulose derivative such as an MC, a HPC, or an HPMC, a starch derivative such as a hydroxypropyl starch or a dextrin, and a natural polysaccharide such as an agar, a galactomannan, a glucomannan, a guar gum, a locust bean gum, a gum acacia (or gum arabic), an arabinogalactan, a tamarind gum, a psyllium seed gum, or a dextran).

The anionic polymer content of the gel-forming layer may be selected from a range in which the anionic polymer can absorb water rapidly to form a gel and inhibit the dissolution of the gel-forming agent and may for example be about 5 to 90% by mass (e.g., about 10 to 80% by mass) in terms of a non-volatile matter. The anionic polymer content of the gel-forming layer may be about 10 to 70% by mass (e.g., about 12 to 50% by mass) and preferably about 15 to 35% by mass (e.g., about 15 to 25% by mass) in terms of a non-volatile matter relative to the whole gel-forming layer.

The gel-forming layer may contain a pharmaceutically acceptable base material or a film-forming agent. The base material (film-forming agent) inhibits cracks of the gel-forming layer, stabilizes the shape of the gel-forming layer, and prevents the separation of the gel from the drug-containing unit. Incidentally, even when the gel-forming layer contains the base material, inflow of water into the drug-containing unit induces formation of foam from the effervescent agent, which triggers rapid separation of the gel from the drug-containing unit.

Examples of the base material (film-forming agent) may include a vinyl-series polymer [for example, a (meth) acrylic polymer, a vinylalcohol-seriespolymer (such as a poly(vinyl alcohol)), a vinylpyrrolidone-series polymer (such as a povidone or a vinyl acetate-vinylpyrrolidone copolymer), a poly(vinyl acetate), and a poly(vinyl acetate phthalate)], a poly(ethylene glycol), and a polysaccharide derived from a plant [for example, a cellulose ether (e.g., an MC, a hydroxymethyl cellulose (HMC), an HEC, an HPC, and an HPMC), a xanthan gum, and a carrageenan]. These components may be used alone or in combination.

Among these film-forming agents, a water-soluble base material [for example, a poly(vinyl alcohol), a vinylpyrrolidone-series polymer, and a cellulose ether] is preferred. Use of the water-soluble base material facilitates the permeation (or infiltration) of water in the gel-forming layer, and the gel-forming layer can rapidly swell in the oral cavity to form a gel. In particular, use of the vinyl alcohol-series polymer (e.g., a poly(vinyl alcohol)) is useful for shielding and masking unpleasant taste and smell of the active ingredient contained in the drug-containing unit.

The base material content of the whole gel-forming layer may be selected from the range of about 20 to 85% by mass (e.g., about 30 to 80% by mass) and may usually be about 50 to 85% by mass and preferably about 60 to 80% by mass (e.g., about 65 to 75% by mass).

The mass ratio of the base material (film-forming agent) relative to the gel-forming agent (e.g., an anionic polymer) may be selected from the range of about 99/1 to 10/90 (e.g., about 90/10 to 15/85, particularly about 85/15 to 20/80) in terms of a solid content, and may usually be about 85/15 to 50/50 (e.g., about 82.5/17.5 to 65/35) and preferably about 80/20 to 70/30, in a ratio of the base material/the gel-forming agent. The ratio of the base material relative to 100 parts by mass of the gel-forming agent may for example be about 50 to 700 parts by mass (e.g., about 100 to 500 parts by mass), preferably about 200 to 400 parts by mass, and more preferably about 250 to 350 parts by mass.

The gel-forming layer can for example be formed as a crosslinked gel-forming layer obtainable from a composition containing the gel-forming agent and a crosslinking agent. The crosslinked gel layer can form a gel having a high strength even in swelling due to water absorption, and having an elasticity and a high slipperiness in the oral cavity. Such a gel facilitates swallowing of the solid preparation and prevents dissolution in the oral cavity.

As the crosslinking agent for the anionic polymer, for example, a polyvalent metal compound can be used. The polyvalent metal compound is not particularly limited to a specific one as far as the compound is a pharmaceutically acceptable metal compound. Such a metal compound may include, for example, a polyvalent metal salt, a polyvalent metal oxide, and a polyvalent metal hydroxide. Examples of the polyvalent metal may include an alkaline earth metal [for example, magnesium and calcium], and metals of the groups 3 to 13 of the Periodic Table of Elements [for example, a metal of the group 8 of the Periodic Table of Elements (e.g., iron), a metal of the group 12 of the Periodic Table of Elements (e.g., zinc), and a metal of the group 13 of the Periodic Table of Elements (e.g., aluminum)].

As these polyvalent metal compounds, for example, there may be mentioned calcium oxide, calcium chloride, magnesium oxide, magnesium chloride, zinc oxide, zinc sulfate, ferric sulfate, iron citrate, aluminum chloride, aluminum hydroxide, aluminum sulfate, aluminum silicate, aluminum phosphate, and an alum compound (for example, aluminum potassium sulfate (potassium alum), ammonium ion (III) sulfate dodecahydrate (ammonium iron alum), and aluminum ammonium sulfate (ammonium alum)). These polyvalent metal compounds may be used alone or in combination. Incidentally, use of a trivalent metal compound increases the degree of crosslinking of the gel-forming agent to improve the physical strength of the gel-forming layer and to prevent the dissolution of the gel-forming agent certainly (or surely).

Regarding the ratio (mass ratio) of the gel-forming agent (e.g., an anionic polymer) relative to the crosslinking agent, the ratio of the crosslinking agent relative to 100 parts by mass of the gel-forming agent is, for example, about 0.1 to 10 parts by mass (e.g., about 0.5 to 7.5 parts by mass), preferably about 1 to 5 parts by mass, and more preferably about 1.5 to 3.5 parts by mass (e.g., about 2 to 3 parts by mass). The crosslinking of the gel-forming agent with the crosslinking agent can retain the form (or shape) of the gel-forming layer while preventing the dissolution of the gel-forming layer. Moreover, a viscosity of a liquid coating composition as a material of the gel-forming layer can be lowered by regulating the ratio of the gel-forming agent and the crosslinking agent to form the gel-forming layer further efficiently.

Further, the ratio of the crosslinking agent relative to 100 parts by mass of the total amount of the base material and the gel-forming agent (e.g., an anionic polymer) may for example be about 0.1 to 2.5 parts by mass, preferably about 0.2 to 1.5 parts by mass (e.g., about 0.25 to 1.2 parts by mass), and more preferably about 0.3 to 1 parts by mass (e.g., about 0.5 to 0.8 parts by mass).

In order to increase the water-absorption speed and gelation speed, the gel-forming layer may contain a water absorption promoter. As the water absorption promoter, there may be used a highly water-soluble component. Examples of the water absorption promoter may include a monosaccharide or a disaccharide (for example, glucose, xylose, mannose, fructose, galactose, sucrose, fruit sugar (or levulose), and white sugar or refined sugar), a polyhydric alcohol [for example, an alkanediol (e.g., propylene glycol), a poly(ethylene glycol) (e.g., a poly(ethylene glycol) having a mass-average molecular weight of 300 to 20000; and a polyoxyethylene polyoxypropylene glycol), and a polyol having three or more hydroxyl groups (a tri- to polyvalent polyol) (e.g., glycerin), a sugar alcohol (e.g., erythritol, sorbitol, xylitol, mannitol, inositol, maltitol, and lactitol)], and an ethylene oxide adduct (e.g., polyoxyl 40 stearate, polyoxyl 45 stearate, polyoxyl 55 stearate, and polyoxyethylene hydrogenated castor oil). These water absorption promoters may be used alone or in combination.

Among these water absorption promoters, the polyhydric alcohol, particularly glycerin, is preferred, since the polyhydric alcohol has an excellent ability to accelerate water absorption and imparts flexibility to the gel to further ease swallowing of the solid preparation. Moreover, the monosaccharide or the disaccharide, the sugar alcohol or the glycerin can also mask the bitterness, acerbity and other unpleasant tastes of the drug.

The water absorption promoter may have a viscosity of about 0.3 to 5.0 mPa·s, preferably about 0.5 to 3.5 mPa·s, and more preferably about 0.6 to 1.8 mPa·s for a 5% by mass aqueous solution at 37°C. The lower the viscosity of the aqueous solution of the water absorption promoter is, the higher the water-absorption speed of the gel-forming layer is.

From the point of view of form (or shape) retention and water absorption (percentage of water absorption) of the gel, the mass ratio of the water absorption promoter relative to 100 parts by mass of the gel-forming agent may be about 1 to 100 parts by mass, preferably about 5 to 75 parts by mass, and more preferably about 10 to 50 parts by mass (e.g., about 25 to 50 parts by mass). Incidentally, when a plurality of water absorption promoters containing glycerin are used, the glycerin content of the whole water absorption promoter may be about 35 to 95% by mass and preferably about 40 to 90% by mass.

The gel-forming layer may contain various optional components, for example, a plasticizer, a masking agent, an antiseptic agent, and a coloring agent, as with the after-mentioned anti-adhesive layer.

It is sufficient that the gel-forming layer covers at least part of the surface of the drug-containing unit (or the surface of the adhesive layer when the solid preparation contains the adhesive layer), particularly, the whole or the most of the surface thereof (for example, about 50 to 100% and preferably about 80 to 100%). The gel-forming layer may cover the surface area of the drug-containing unit or that of the adhesive layer uniformly or nonuniformly (scatteringly in a polygonal pattern such as quadrilateral pattern, a circular pattern, or a grid pattern). The gel-forming layer usually covers the whole of the drug-containing unit or that the adhesive layer (in the above-mentioned embodiment, at least upper and under surfaces).

The thickness of the gel-forming layer may be selected from the range of, for example, about 1 to 1000 μm (e.g., about 3 to 700 μm) and may be about 5 to 500 μm, and preferably about 7 to 250 μm (e.g., about 10 to 100 μm). Even a layer having a thickness of about 5 to 50 μm (e.g., about 10 to 30 μm) performs a sufficient function as the gel-forming layer. Incidentally, when the gel-forming layer is prepared, a plurality of thin gel-forming layers [each having a thickness of not more than 10 μm (e.g., 1 to 10 μm, preferably 2 to 9 μm, and more preferably 3 to 8 μm)] may be laminated (or layered) to form a gel-forming layer having a predetermined thickness, thereby accelerating the gelation speed, according to a method described in Japanese Patent Application Laid-Open Publication No. 2008-37794.

[Anti-adhesive layer (surface layer)]

The anti-adhesive layer (surface layer) is not necessarily required, and covering of the gel-forming layer with the anti-adhesive layer (surface layer) directly or indirectly is advantageous to prevention of adhesion of the solid preparation to the inner wall of the oral cavity by dissolving the anti-adhesive layer in water. Therefore, the preparation provided with the anti-adhesive layer (surface layer) covering the gel-forming layer is useful for extensive improvement of medication compliance from infants to elderly people.

As the component of the anti-adhesive layer (surface layer), for example, there may be mentioned a water-soluble polymer [e.g., a cellulose derivative [an alkyl cellulose (such as an MC), a hydroxyalkyl cellulose (such as an HEC, an HPC, or an HPMC), and a carboxymethyl cellulose (such as a CMC or a CMC-sodium)], a poly(ethylene glycol), a polyoxyethylene polyoxypropylene glycol, a poly(vinyl alcohol), an ethylene oxide adduct of a higher fatty acid or polyhydric alcohol fatty acid ester (e.g., a polyoxyethylene stearate, a polyoxyethylene sucrose fatty acid ester, a polyoxyethylene sorbitan fatty acid ester, and a polyoxyethylene hydrogenated castor oil), a natural polysaccharide (such as a gum acacia (or gum arabic)), and a protein (such as a gelatin)]; a saccharide [e.g., erythritol, sorbitol, xylitol, mannitol, inositol, maltitol, lactitol, glucose, xylose, mannose, fructose, galactose, lactose, white sugar or refined sugar, maltose, glucose, sucrose, and fruit sugar (or levulose)]; and a polyhydric alcohol (e.g., propylene glycol and glycerin). These components may be used alone or in combination. Among these components, a water-soluble polymer [for example, a cellulose derivative (e.g., an alkyl cellulose (such as an MC) and a hydroxyalkyl cellulose (such as an HEC, an HPC, or an HPMC)), a poly (ethylene glycol), are a poly (vinyl alcohol)] is practically used.

The preferred anti-adhesive layer (surface layer) contains a water-soluble cellulose ether and an anionic polymer and prevents adhesion of the solid preparation to the inner wall of the oral cavity. Such an anti-adhesive layer is dissolved by a small quantity of water or moisture (e.g., saliva) and more certainly forms an aqueous liquid coat around the gel formed from the gel-forming layer due to water absorption and swelling. Accordingly, the direct adhesion (attachment) of the gel-forming layer to the inner wall of the oral cavity can be prevented, and even if part of the gel-forming layer is adhered, the gel-forming layer is easily separated from the inner wall. Moreover, for oral administration, the adhesion of the solid preparation to the inner wall of the oral cavity over a longer period of time can certainly be prevented.

The water-soluble cellulose ether may include an alkyl cellulose [for example, a methyl cellulose (MC)], a hydroxyalkyl cellulose [for example, a hydroxyethyl cellulose (HEC) and a hydroxypropyl cellulose (HPC)], and a hydroxyalkylalkyl cellulose [for example, a hydroxyethylmethyl cellulose (HEMC) and a hydroxypropylmethyl cellulose (HPMC) (e.g., HPMC2208, HPMC2906, and HPMC2910)], a carboxymethyl cellulose [e.g., a CMC, a CMC-sodium, and a carboxymethyl cellulose], and others. These cellulose ethers may be used alone or in combination.

Among these water-soluble cellulose ethers, the preferred one includes at least one member selected from the group consisting of a methyl cellulose, a hydroxyethyl cellulose, a hydroxyethylmethyl cellulose, a hydroxypropyl cellulose, and a hydroxypropylmethyl cellulose. Incidentally, for the water-soluble cellulose ether, the hydroxyalkyl cellulose (e.g., an HEC and an HPC), the hydroxyalkylalkyl cellulose (e.g., a hydroxy$C_{2-3}$alkylmethyl cellulose such as an HEMC or an HPMC), and the alkyl cellulose (e.g., an MC) seems to have an action preventing the adhesion of the solid preparation to the inner wall of the oral cavity in descending order of degree.

In the hydroxyalkylmethyl cellulose, the content of ether groups derived from all hydroxyl groups of the cellulose is not particularly limited to a specific one. In order to prevent the adhesion of the solid preparation to the inner wall of the oral cavity, it is preferable that the average substitution degree of methyl group be larger and the average substitution degree of hydroxyalkyl group be smaller. Concretely, the methoxy group content (substitution ratio) may for example be about 5 to 40%, preferably about 10 to 35%, and more preferably about 15 to 30%; and the hydroxyalkoxy group content (substitution ratio) may for example be about 0.1 to 20%, preferably about 1 to 15%, and more preferably about 2 to 10%. The ratio of the methoxy group content (substitution ratio) relative to the hydroxyalkoxy group content (substitution ratio) may for example be about 90/10 to 50/50, preferably about 85/15 to 60/40, and more preferably about 80/20 to 70/30, as the methoxy group/the hydroxyalkoxy group.

Among the hydroxyalkylmethyl celluloses, an HPMC is preferred. Representative examples of the HPMC may include HPMC2208, HPMC2906, and HPMC2910, and HPMC2910 is particularly preferred.

The viscosity of the water-soluble cellulose ether for a 2% by mass aqueous solution at 20°C may be not more than 50 mPa·s, preferably not more than 40 mPa·s, and more preferably about 1 to 30 mPa·s. Probably or presumably because of more rapid dissolution in a small quantity of water (e.g., saliva) and formation of a lower viscous aqueous liquid coat, a water-soluble cellulose ether having a lower viscosity can effectively prevent the adhesion of the solid preparation to the inner wall of the oral cavity.

The content of the water-soluble cellulose ether of the whole anti-adhesive layer may be selected from the range of about 20 to 99% by mass (e.g., about 30 to 98% by mass) and may usually be about 50 to 95% by mass (e.g., about 60 to 95% by mass) and preferably about 70 to 90% by mass (e.g., about 75 to 90% by mass).

As far as the anionic polymer can be dissolved in water (e.g., saliva) in an environment of the oral cavity, there are no particular limitations thereon. For example, the anionic polymer may include the water-soluble polymer (an anionic polymer such as a carboxy group-containing polymer, a sulfonic acid group-containing polymer, or a phosphoric acid group-containing polymer) as described as the gel-forming agent for the gel-forming layer. The anionic polymer may form, for example, a salt with an inorganic base [e.g., an alkali metal (such as sodium or potassium) and ammonia] or an organic base [e.g., monoethanolamine, diethanolamine, triethanolamine, and dimethylaminoethanol]. The preferred anionic polymer includes the above-mentioned carboxy group-containing polymer, particularly a (meth) acrylic acid-series polymer comprising a (meth)acrylic acid unit as an essential polymerizable component [a homo- or copolymer of (meth) acrylic acid].

The monomer copolymerizable with (meth)acrylic acid may include the copolymerizable monomer described in the gel-forming agent and may be used alone or in combination. For the (meth) acrylic acid-series polymer, the ratio (mass ratio) of the (meth) acrylic acid (or a salt thereof) relative to the copolymerizable monomer is not particularly limited to a specific one as far as the (meth) acrylic acid-series polymer is water-soluble, and for example, the ratio is the same as that described in the gel-forming agent.

As the (meth)acrylic acid-series polymer, there may be mentioned an acrylic acid-series polymer [for example, a polyacrylic acid, an acrylic acid-alkyl acrylate copolymer (e.g., an acrylic acid-methyl acrylate copolymer and an acrylic acid-ethyl acrylate copolymer), and an acrylic acid-alkyl methacrylate copolymer (e.g., acrylic acid-methyl methacrylate and acrylic acid-ethyl methacrylate)], and a methacrylic acid-seriespolymer (e.g., methacrylic acid-alkyl acrylate copolymer such as a methacrylic acid-methyl acrylate copolymer or a methacrylic acid-ethyl acrylate copolymer). These (meth) acrylic acid-series polymers may be used alone or in combination. The viscosity of the anionic polymer for a 0.2% by mass aqueous solution is usually the same as the viscosity of the aqueous solution of the above-mentioned gel-forming agent.

Representative examples of the (meth)acrylic acid-series polymer may include a carboxyvinyl polymer (trade name: CARBOPOL), a poly (sodium acrylate), a partially neutralized product of a polyacrylic acid, a methacrylic acid-n-butyl acrylate copolymer, and a methacrylic acid copolymer LD (trade name: EUDRAGIT L-30D55). Among these (meth)acrylic acid-series polymers, the preferred one includes an acrylic acid-series polymer obtained by using acrylic acid as a main monomer, particularly a carboxyvinyl polymer (e.g., CARBOPOL and HIVISWAKO exemplified in the above-mentioned gel-forming agent).

The anionic polymer content of the anti-adhesive layer may be selected from the range in which the anti-adhesive layer can rapidly absorb water to form a liquid coat while preventing the adhesion of the solid preparation to the inner wall of the oral cavity, and may for example be about 0.1 to 50% by mass (e.g., about 1 to 30% by mass) in terms of a solid content or a non-volatile matter. The anionic polymer content of the whole anti-adhesive layer may be about 1 to 25% by mass (e.g., about 2 to 20% by mass) and preferably about 3 to 17% by mass (e.g., about 5 to 15% by mass) in terms of a non-volatile matter.

Depending on the species of the water-soluble cellulose ether and anionic polymer, when the water-soluble cellulose ether and the anionic polymer are the same species as the base material of the gel-forming layer and the gel-forming agent, respectively, the ratio of the water-soluble cellulose ether relative to the anionic polymer in the anti-adhesive layer is usually larger than the ratio of the base material relative to the gel-forming agent (an anionic polymer such as a carboxyvinyl polymer) of the gel-forming layer. The mass ratio of the water-soluble cellulose ether relative to the anionic polymer in terms of a solid content may be selected from the range of about 99.9/0.1 to 75/25 (e.g., about 99/1 to 80/20), and may usually be about 99.9/0.1 to 85/15 (e.g., about 99/1 to 85/15) and preferably about 95/5 to 85/15 (e.g., about 92/18 to 87/13), in a ratio of the water-soluble cellulose ether/the anionic polymer. The ratio of the water-soluble cellulose ether relative to 100 parts by mass of the anionic polymer may for example be about 100 to 2000 parts by mass (e.g., about 200 to 1500 parts by mass), preferably about 300 to 1200 parts by mass (e.g., about 500 to 1000 parts by mass), and more preferably about 600 to 900 parts by mass.

The anti-adhesive layer sometimes has an excessively higher or lower viscosity depending on the species or molecular weight of the water-soluble cellulose and that of the anionic polymer, so that the anti-adhesive layer sometimes fails to show the function as an anti-adhesive layer. Moreover, when a liquid coating composition for forming the anti-adhesive layer has an excessively higher viscosity, the anti-adhesive layer cannot be formed smoothly in some cases. Thus, the anti-adhesive layer may contain a viscosity modifier for adjusting the viscosity, particularly a viscosity reducing agent or an auxiliary. As the viscosity reducing agent, a metal salt highly reducing the viscosity of the solution (for example, an alkali metal salt and an alkaline earth metal salt) isusedpractically. The amount of the viscosity reducing agent relative to 100 parts by mass of the total amount of the water-soluble cellulose ether and the anionic polymer may for example be selected from the range of about 0 to 200 parts by mass and may usually be about 1 to 100 parts by mass, preferably about 5 to 50 parts by mass, and more preferably about 10 to 30 parts by mass.

Incidentally, the polyvalent metal salt (an alkaline earth metal salt, a tri- to polyvalent metal salt) may function as a crosslinking agent for the anionicpolymer. When such a polyvalent metal salt is used as the viscosity reducing agent for the anti-adhesive layer, the amount of the polyvalent metal salt is smaller than the amount of the crosslinking agent relative to 100 parts by mass of the total amount of the base material and the gel-forming agent in the gel-forming layer. The amount of the polyvalent metal salt in the anti-adhesive layer may for example be about 0 to 2 parts by mass (e.g., about 0.01 to 1.5 parts by mass), preferably about 0.05 to 1 parts by mass, and more preferably about 0.1 to 0.5 parts by mass (e.g., about 0.2 to 0.4 parts by mass) relative to 100 parts by mass of the total amount of the water-soluble cellulose ether and the anionic polymer (e.g., a carboxyvinyl polymer). Incidentally, the ratio of the polyvalent metal salt relative to 100 parts by mass of the anionic polymer (e.g., a carboxyvinyl polymer) may for example be about 0.1 to 10 parts by mass (e.g., about 0.5 to 7.5 parts by mass), preferably about 1 to 5 parts by mass, and more preferably about 1.5 to 3.5 parts by mass (e.g., about 2 to 3 parts by mass).

The anti-adhesive layer may contain the various additives as described above, such as the water absorption promoter (for example, glycerin), the masking agent for masking the taste or smell of the active ingredient, the plasticizer (for example, glycerin triacetate, diethyl phthalate, and triethyl citrate), the antiseptic agent or the preservative (for example, methyl hydroxybenzoate, propyl hydroxybenzoate, sodium edetate, potassium sorbate, and sodium dehydroacetate), the antioxidant (such as ascorbic acid or tocopherol acetate), and the coloring agent (for example, titanium oxide, and edible lake coloring agent). The masking agent may include an acidifier or an acidulant (e.g., citric acid, tartaric acid, and fumaric acid), a sweetening agent (e.g., saccharin, glycyrrhizinic acid, aspartame, stevioside, acesulfame potassium, and a saccharide), an algefacient (e.g., menthol, mentha oil, peppermint, and spearmint), a natural or synthetic flavoring agent (or perfume), and others. Among these masking agents, a saccharide (a sugar such as lactose, white sugar or refined sugar, glucose, or sucrose, a sugar alcohol such as mannitol, sorbitol, or xylitol) is preferred.

These components may also be used alone or in combination. The amount of these components may be not more than 20 parts by mass (e.g., about 0.01 to 15 parts by mass, preferably about 0.05 to 10 parts by mass, and more preferably about 0.1 to 10 parts by mass) relative to 100 parts by mass of the total amount of the water-soluble cellulose ether and the anionic polymer (in terms of a solid content) .

It is sufficient that the anti-adhesive layer covers at least part of the surface of the gel-forming layer (for example, not less than 50% of the surface area of the gel-forming layer (e.g., about 50 to 100%, preferably about 87 to 100%, and more preferably about 90 to 100%)). The anti-adhesive layer practically covers the whole of the gel-forming layer or at least upper and under surfaces thereof. The anti-adhesive layer may cover the surface of the gel-forming layer uniformly or nonuniformly (e.g., scatteringly in a polygonal pattern (e.g., quadrilateral pattern), a circular pattern, or a grid pattern).

In order to easily permeate even a small quantity of water (such as saliva) into the anti-adhesive layer, the thickness of the anti-adhesive layer may be not more than 50 $\mu$m (e.g., about 1 to 50 $\mu$m, preferably about 5 to 45 $\mu$m, and more preferably about 10 to 40 $\mu$m).

The total thickness of the gel-forming layer and the anti-adhesive layer may for example be about 5 to 1000 $\mu$m, preferably about 10 to 500 $\mu$m (e.g., about 15 to 250 $\mu$m), and more preferably about 20 to 100 $\mu$m (e.g., about 25 to 75 $\mu$m). Moreover, the thickness ratio of the gel-forming layer relative to the anti-adhesive layer may be selected from the range of about 5/95 to 95/5 (e.g., about 10/90 to 90/10) and may be about 15/85 to 50/50 and more preferably about 20/80 to 40/60 (e.g., about 20/80 to 30/70), as the gel-forming layer/the anti-adhesive layer. By controlling the thickness ratio of the gel-forming layer relative to the anti-adhesive layer, the gel-forming layer rapidly absorbs water through the anti-adhesive layer and swells to form a gel layer having a significantly improved slipperiness in a short period of time, and the anti-adhesive layer can form an aqueous liquid coat as the surface layer. Probably due to such a structure, the solid preparation (solid preparation for oral administration) can easily be swallowed without adhesion to the inner wall of the oral cavity even in absence of water and improve the ease (or easiness) of taking the preparation significantly.

[Physiologically(or pharmaceutically) acceptable effervescent agent]

The physiologically (or pharmaceutically) acceptable effervescent agent can be contained in the drug-containing unit and/or the intermediate layer. When the effervescent agent is contained in the drug-containing unit and/or the intermediate layer, the effervescence of the effervescent agent allows a gel formed around the drug-containing unit by water absorption and swelling of the gel-forming layer to be disintegrated rapidly, and the elution property of the drug from the solid preparation (for example, the elution property of the drug from the solid preparation at an initial stage of disintegration of the solid preparation) is improvable. Since the effervescence of the effervescent agent becomes notable several minutes after contact of the solid preparation with an aqueous fluid (such as saliva or gastric juice), the drug is not eluted in the oral cavity when administered, so that the solid preparation excellently masks the taste of the drug or the like. In addition, the effervescent agent becomes notable after the solid preparation reaches the stomach, and a gel formed around the drug-containing unit by water absorption and swelling of the gel-forming layer is allowed to be disintegrated rapidly, so that the elution of the drug from the drug-containing unit can be promoted. Incidentally, incorporation of the effervescent agent into the intermediate layer can reduce an influence of the effervescent agent on the drug, which is advantageous from the aspect of the stability of the preparation, and others.

The effervescent agent is not particularly limited to a specific one as far as the agent is a component which is allowed to react with water to produce gas (such as carbon dioxide). For example, the effervescent agent may include a salt of an anionic component with a cationic component. As the anionic component, there may be mentioned an inorganic acid (such as carbonic acid), an organic acid (such as tartaric acid or citric acid), and others. As the cationic component, there may be mentioned an alkali metal, an alkaline earth metal, ammonia, an others.

Representative examples of the effervescent agent may include an alkali metal carbonate (e.g., sodium hydrogencarbonate, potassium hydrogencarbonate, sodium carbonate, and potassium carbonate), an alkaline earth metal carbonate (e.g., calcium carbonate), and ammonium carbonate.

These effervescent agents may be used alone or in combination. Among these effervescent agents, the preferred one may include a salt of at least one selected from the group consisting of an alkali metal, an alkaline earth metal, and ammonia, each of which is a carbonate, a hydrogencarbonate (bicarbonate), or a sesquicarbonate. An alkali metal carbonate or an alkali metal hydrogencarbonate (e.g., sodium hydrogencarbonate) is particularly preferred.

The ratio of the effervescent agent contained in the drug-containing unit may suitably be selected according to the volume of the drug-containing unit, or others. The ratio of the effervescent agent contained in the drug-containing unit may be selected from the range of about 0.001 to 200 parts by mass relative to 1 part by mass of the drug, or may be about 0.1 to 160 parts by mass (e.g., about 1 to 150 parts by mass), preferably about 3 to 80 parts by mass (e.g., about 4 to 70 parts by mass), and more preferably 5 to 60 parts by mass (particularly about 6 to 40 parts by mass) relative to 1 part by mass of the drug.

The ratio of the effervescent agent contained in the intermediate layer may be selected from the range of about 0.001 to 100 parts by mass relative to 1 part by mass of the drug, or may be about 0.01 to 50 parts by mass (e.g., about 0.05 to 30 parts by mass), preferably about 0.1 to 25 parts by mass (e.g., about 0.5 to 20 parts by mass), more preferably about 1 to 15 parts by mass (particularly about 5 to 10 parts by mass) relative to 1 part by mass of the drug. Moreover, the ratio of the effervescent agent contained in the intermediate layer may be selected from the range of about 0.001 to 100 parts by mass relative to 100 parts by mass of the base material (adhesive agent) contained in the intermediate layer, or may be about 0.1 to 50 parts by mass (e.g., about 0.5 to 40 parts by mass), preferably about 1 to 35 parts by mass (e.g., about 5 to 30 parts by mass), and more preferably about 10 to 25 parts by mass (particularly about 15 to 20 parts by mass) relative to 100 parts by mass of the base material (adhesive agent) contained in the intermediate layer.

Combination use of the effervescent agent with the disintegrant (for example, an acidic disintegrant) described in the paragraph of the drug-containing unit can improve the effervescence of the effervescent agent. The acidic (or acidic group-containing) disintegrant may include, for example, a cellulose or starch having an acidic group (e.g., a carboxy$C_{1-2}$alkyl group such as carboxyl group or carboxymethyl group), e.g., a carboxymethyl cellulose, a carboxymethyl starch, and a cellulose acetate phthalate. Such a disintegrant (for example, an acidic disintegrant) also has a function as effervescent auxiliary. The disintegrants (for example, an acidic disintegrant) may be used alone or in combination. The preferred disintegrant may include at least one member selected from the group consisting of a carboxymethyl cellulose, a carboxymethyl starch, and a cellulose acetate phthalate. A carboxymethyl cellulose or starch (particularly a carboxymethyl cellulose) is more preferred.

The cellulose or starch having acidic groups (e.g., a carboxymethyl cellulose) may have acidic groups (e.g., carboxymethyl group) at an average degree of substitution (or an average degree of etherification, DS) of, for example, about 0.3 to 0.7, preferably about 0.35 to 0.65, and more preferably about 0.4 to 0.6. The term "average degree of substitution" means the average value of substitution of acidic groups for hydroxyl groups at 2-, 3-, and 6-positions of glucose units in a cellulose or starch. The maximum value is 3.

The pH of a mixture containing 1% by weight of the disintegrant in water (aqueous dispersion or aqueous solution) is not particularly limited to a specific one. In terms of improvement of the effervescence of the effervescent agent, or others, the pH of the mixture may be about 2.0 to 6.5 (e.g., about 2.5 to 6.0), preferably about 3.0 to 5.5, and more preferably about 3.5 to 5.

The ratio of the disintegrant (for example, an acidic disintegrant) may suitably be selected according to the elution property of the drug. The ratio of the disintegrant may be selected from the range of about 0 to 100 parts bymass (e.g., about 1 to 100 parts by mass) relative to 100 parts by mass of the effervescent agent, or may for example be about 10 to 80 parts by mass (e.g., about 15 to 75 parts by mass), preferably about 20 to 70 parts by mass (e.g., about 25 to 65 parts by mass), and more preferably about 30 to 60 parts by mass (e.g., about 35 to 55 parts by mass, particularly about 40 to 50 parts by mass) relative to 100 parts by mass of the effervescent agent. Incidentally, the above-mentioned ratio may be a ratio of the disintegrant contained in the drug-containing unit relative to the effervescent agent contained in the drug-containing unit, a ratio of the disintegrant contained in the intermediate layer relative to the effervescent agent contained in the intermediate layer, or a ratio of the disintegrant contained in the whole solid preparation relative to the effervescent agent contained in the whole solid preparation.

Incidentally, when the effervescent agent or the disintegrant is contained in both drug-containing unit and intermediate layer, the species of the effervescent agent or the disintegrant contained in the drug-containing unit may be different from that contained in the intermediate layer or may be the same as that contained in the intermediate layer.

[Physiologically(or pharmaceutically) acceptable electrolyte]

The physiologically (pharmaceutically) acceptable electrolyte can be contained in the drug-containing unit and/or the intermediate layer, and if necessary, may be incorporated into the gel-forming layer and/or the anti-adhesive layer. Incidentally, the electrolyte contained in the intermediate layer can reduce adverse affects on the drug, and it is advantageous in view of the stability of the preparation and others.

It is sufficient that the electrolyte is a component at least partly soluble in water and dissociable into ions thereof regardless of solubility. The electrolyte may be easily soluble in water or hardly or sparingly soluble in water. Moreover, the electrolyte may be either a strong electrolyte or a weak electrolyte. The electrolyte generates a counter ion and inhibits adsorption on or ionic bonding of the cationic drug to the anionic polymer.

The species of the pharmaceutically acceptable electrolyte may be selected from various salts or compounds of a cationic component and an anionic component depending on the basicity of the drug, the acidity of the anionic polymer, and others. The cationic component of the electrolyte may include cations corresponding to the following: ammonium, a metal {a monovalent metal [e.g., an alkali metal (e.g., sodium and potassium)], a polyvalent metal [metals of the groups 2 to 13 of the Periodic Table of Elements such as an alkaline earth metal (e.g., magnesium and calcium), a metal of the group 8 of the Periodic Table of Elements (e.g., iron), a metal of the group 12 of the Periodic Table of Elements (e.g., zinc), and a metal of the group 13 of the Periodic Table of Elements (e.g., aluminum)]}, and others. As the anionic component, there may be mentioned anions corresponding to the following: an inorganic acid [e.g., hydrochloric acid, hydrobromic acid, sulfuric acid, phosphoric acid, nitric acid, silicic acid, and boric acid], an organic acid [e.g., a carboxylic acid (e.g., an aliphatic saturated monocarboxylic acid such as acetic acid, propionic acid, or butyric acid; an aliphatic saturated dicarboxylic acid such as malonic acid, succinic acid, glutaric acid, or adipic acid; an aliphatic unsaturated carboxylic acid such as maleic acid or fumaric acid; and an aromatic carboxylic acid such as benzoic acid), a hydroxycarboxylic acid (an aliphatic saturated monocarboxylic acid such as lactic acid; an aliphatic saturated dicarboxylic acid such as malic acid or tartaric acid; an aliphatic saturated tricarboxylic acid such as citric acid or isocitric acid; and an aromatic carboxylic acid such as salicylic acid), a sulfonic acid (e.g., methanesulfonic acid, benzenesulfonic acid, and toluenesulfonic acid), and amino acid], and others.

Representative examples of the electrolyte may include an alkali metal compound [for example, a halide (a chloride such as sodium chloride or potassium chloride) ; an inorganic acid salt (a sulfate such as sodium sulfate or potassium sulfate; a phosphate (for example, a sodium phosphate such as sodium monohydrogenphosphate, sodium dihydrogenphosphate, or trisodium phosphate, and a potassium phosphate such as dipotassium phosphate or potassium dihydrogenphosphate), and an organic acid salt (e.g., sodium acetate, sodium fumarate, sodium lactate, sodium citrate, sodium tartrate, sodium potassium tartrate, and potassium hydrogentartrate)], an alkaline earth metal compound [for example, a halide (a chloride such as magnesium chloride or calcium chloride); an inorganic acid salt (a sulfate such as calcium sulfate or magnesium sulfate; a phosphate such as calcium hydrogenphosphate, calcium monohydrogenphosphate, calcium dihydrogenphosphate, or manganese phosphate; and a silicate such as magnesium silicate), and an organic acid salt (such as calcium acetate or calcium lactate)], a polyvalent metal salt [for example, a halide (a chloride such as aluminum chloride or zinc chloride) ; an inorganic acid salt (a sulfate such as aluminum sulfate, aluminum potassium sulfate, or zinc sulfate; a phosphate such as aluminum phosphate; a silicate such as aluminum silicate), an organic acid salt (such as aluminum acetate, zinc acetate, or aluminum lactate)], and others. These electrolytes may be a hydrate or a double salt (or complex).

These electrolytes may be used alone or in combination. Among these electrolytes, the following electrolyte is practically used: an alkali metal compound (particularly a sodium compound and a potassium compound) and an alkaline earth metal compound (particularly a calcium compound and a magnesium compound), for example, a chloride (an alkali metal chloride such as sodium chloride or potassium chloride, an alkaline earth metal chloride such as calcium chloride or magnesium chloride), a phosphate (an alkali metal phosphate such as sodium monohydrogenphosphate, sodium dihydrogenphosphate, or dipotassium phosphate, an alkaline earth metal phosphate such as calcium hydrogenphosphate, calcium monohydrogenphosphate, or calcium dihydrogenphosphate), an organic acid salt [an alkali metal carboxylate, e.g., an alkali metal acetate (such as sodium acetate or potassium acetate); an alkali metal hydroxycarboxylate, e.g., an alkali metal lactate (such as sodium lactate), an alkali metal citrate (such as sodium citrate, sodium dihydrogencitrate, or disodium citrate), an alkali metal tartrate (such as sodium tartrate, sodium potassium tartrate, or potassium hydrogentartrate), an alkaline earth metal acetate (such as calcium acetate), an alkaline earth metal hydroxycarboxylate, e.g., an alkaline earth metal lactate (such as calcium lactate), and an alkaline earth metal citrate (such as calcium citrate)], particularly, a chloride, a phosphate, and the like (in particular, a phosphate). These electrolytes may be water-insoluble. A water-soluble electrolyte is advantageous. Incidentally, in order to improve the elution property (or dissolution) of the cationic drug, it is advantageous that a polyvalent cation (e.g., an alkaline earth metal compound) rather than a monovalent cation (e.g., an alkali metal compound) is used.

Considering from the easy water-solubility, the molecular weight of the electrolyte is not more than 1000 g/mol and preferably about 50 to 500 g/mol.

The amount of the electrolyte is not particularly limited to a specific one. For example, when the drug is a cationic drug, the amount of the electrolyte maybe selected from the range preventing the adsorption of the cationic drug to the anionic polymer. The amount of the electrolyte may for example be about 1 to 5000 parts by mass (e.g., about 10 to 3000 parts by mass), preferably about 25 to 2500 parts by mass (e.g., about 50 to 2000 parts by mass), and more preferably about 75 to 1700 parts by mass (e.g., about 100 to 1500 parts by mass) relative to 100 parts by mass of the drug (e.g., the cationic drug). Moreover, the molar ratio of the electrolyte relative to 1 mol of the drug (e.g., the cationic drug) may be about 0.1 to 150 mol (e.g., about 0.5 to 125 mol), preferably about 1 to 100 mol (e.g., about 2 to 100 mol), and more preferably about 3 to 75 mol (e.g., about 5 to 50 mol).

Incidentally, when the electrolyte is contained in both drug-containing unit and intermediate layer, the species of the electrolyte contained in the drug-containing unit maybe different from that contained in the intermediate layer or may be the same as that contained in the intermediate layer. The ratio of the electrolyte content by percentage (mass ratio) of the drug-containing unit relative to the electrolyte content by percentage (mass ratio) of the intermediate layer may suitably be selected according to the stability of the drug, and the like. For example, the ratio of the electrolyte content (mass ratio) of the drug-containing unit relative to the electrolyte content (mass ratio) of the intermediate layer may for example be about 1/99 to 99/1, preferably about 5/95 to 95/5, and more preferably about 10/90 to 90/10 in a ratio of the former/the latter.

[Ratio of effervescent agent relative to electrolyte]

According to the present invention, in order to improve the elution property of the drug, the effervescent agent and the electrolyte can be used in combination. The electrolyte may be contained in a layer free from the effervescent agent, or maybe contained in a layer containing the effervescent agent to coexist with the effervescent agent. The ratio (mass ratio) of the effervescent agent relative to the electrolyte may for example be selected from the range of about 100/0 to 1/99 or may be about 70/30 to 20/80 (e.g., about 70/30 to 30/70), preferably about 65/35 to 25/75 (e.g., about 65/35 to 35/65), and more preferably 60/40 to 30/70 (e.g., about 60/40 to 40/60). Incidentally, the above-mentioned ratio may be a ratio of the effervescent agent contained in the drug-containing unit relative to the electrolyte contained in the drug-containing unit, a ratio of the effervescent agent contained in the intermediate layer relative to the electrolyte contained in the intermediate layer, or a ratio of the effervescent agent contained in the whole solid preparation relative to the electrolyte contained in the whole solid preparation.

[Shape of solid preparation]

It is sufficient that the solid preparation comprises the drug-containing unit and the gel-forming layer, and the adhesive layer is not necessarily required. Moreover, the solid preparation does not necessarily comprise the anti-adhesive layer. In order to prevent the contact of the drug of the drug-containing unit with the effervescentagentorthe electrolyte,thesolid preparation may have the drug-containing unit covered (or coated) with a covering layer, for example, a gastric-soluble coating layer or an enteric coating layer. Further, if necessary, an enteric coating layer, a gastric-soluble coating layer, or other layers may be formed at an appropriate interlayer of the intermediate layer, the gel-forming layer, and the anti-adhesive layer. The enteric component may include, for example, an enteric base material described in the above-mentioned drug-containing unit. The gastric-soluble component may include, for example, a gastric-soluble base material described in the above-mentioned drug-containing unit.

The solid preparation (or solid preparation for oral administration) of the present invention may be in the form corresponding to the drug-containing unit or in the form in which the gel-forming layer and the anti-adhesive layer are extended from the periphery of the drug-containing unit. Moreover, the solid preparation of the present invention may be a film-shaped preparation in the form of a flat shape or a discoid shape, for example, a flat or discoid preparation having the drug-containing unit enclosed (or wrapped) with a film- or sheet-like covering layer(s). The plane shape of the film-shaped preparation may for example be a polygon (e.g., a quadrilateral), a circle, and an ellipse. According to the solid preparation of the present invention, the gel-forming layer and the anti-adhesive layer improves the slipperiness in the oral cavity by even a small quantity of water. Therefore, even when the film-shaped preparation has a large flat-surface area, the preparation can easily be swallowed. The area of the flat surface of the film-shaped preparation is not particularly limited to a specific one, and may be about 0.01 to 10 cm$^2$ (e.g., about 0.05 to 9 cm$^2$, preferably about 0.1 to 8 cm$^2$, and about more preferably 0.5 to 7 cm$^2$).

Incidentally, the surface of the solid preparation may be embossed, if necessary.

[Process for producing solid preparation]

The solid preparation of the present invention may be prepared by covering the drug-containing unit with the gel-forming layer, if necessary, through the intermediate layer, and the gel-forming layer may be covered with the anti-adhesive layer. The drug-containing unit can be prepared using the active ingredient, the effervescent agent, and the additive according to a conventional manner (such as granulation or tableting), as described above. Moreover, each layer of the solid preparation can be produced by each applying a coating composition corresponding to each layer to the drug-containing unit sequentially. Each of the coating compositions corresponding to each layer can be prepared by dispersing or dissolving constituents of each layer (for example, the anti-adhesive layer) in a liquid medium such as water (e.g., a purified water) or a lower alcohol (e.g., ethanol), optionally an organic solvent. Incidentally, if necessary, the resulting coating composition (liquid coating composition or coating agent) may be defoamed.

Depending on the dosage form, a method for coating the drug-containing unit with the coating composition may include, for example, apancoating, afluidizedbedcoating, a tumbling coating, and a tumbling fluidized bed coating.

For example, coating (applying), spraying, and impregnation or dipping may be used for coating the drug-containing unit with the coating composition. Incidentally, each coating composition may be coated (or applied) successively after drying or without drying.

For the preparation of the solid preparation of the present invention, there may be used lamination or stacking of each layer to the drug-containing unit by flow-casting, coating (applying), or other means. For example, the solid preparation of the present invention may be prepared by a process which comprises an optional step for applying an anti-adhesive composition (coating agent) to a releasable (separable) substrate to form an anti-adhesive layer (an anti-adhesive layer forming step), a step for laminating a gel-forming layer on the anti-adhesive layer (a gel-forming layer laminating step), and a step for laminating an intermediate layer on the gel-forming layer (an intermediate layer laminating step), and a step for interposing a drug-containing unit between two laminates prepared through these steps and adhering (or bonding) these laminates (an adhering step).

The releasable substrate is not particularly limited to a specific one, and, for example, a glass plate, a plastic film, and a release sheet may be used. If necessary, these releasable substrates may be embossed by a conventional manner.

The anti-adhesive layer, the gel-forming layer, and the intermediate layer can be formed by coating each liquid coating composition on the releasable substrate using a conventional film-forming method (for example, a method using coating (applying) such as flow-casting, or spraying) . Incidentally, the intermediate layer may be formed by coating the gel-forming layer partly (for example, by coating a surrounding region of the drug-containing unit in the resulting solid preparation). Moreover, the anti-adhesive layer is not essentially formed on the whole surface of the gel-forming layer. In order to form the aqueous liquid coat and the gel layer uniformly and improve the ease of swallowing the preparation, the whole surface of the gel-forming layer is practically coated with the anti-adhesive layer.

In the adhering step, a pair of laminates can be adhered (bonded) to each other while interposing the drug-containing unit between these laminates with the gel-forming layers (or intermediate layers) facing each other. The drug-containing unit can be arranged at a predetermined position with the use of a method for positioning the solid preparation (such as a powdered preparation or a tablet) containing the drug at a predetermined site or area (a central site or area of the solid preparation of the present invention), coating (applying), spraying, dropping, ink-jetting, screen-printing, or others. Incidentally, when an embossed releasable substrate having the gel-forming layer (or intermediate layer) is used, the drug-containing unit may be placed in a recessed area formed in the gel-forming layer (or intermediate layer).

When a heat (or thermal) adhesive is used for the intermediate layer, thermal adhesion (or heat-sealing) or other means can be utilized as a method for adhering the laminates. The temperature of the thermal adhesion may for example be about 70 to 150°C (e.g., about 75 to 140°C, preferably about 80°C to 130°C, and more preferably about 85 to 120°C).

The solid preparation can be produced by adhering the periphery of the drug-containing unit to prepare a laminate (or laminated product) having the above layers, and then punching out the periphery of the drug-containing unit in a predetermined shape (e.g., a circular shape, an elliptical shape, and a polygonal shape) depending on the shape of the drug-containing unit.

Moreover, the solid preparation of the present invention can also be obtained by covering a drug-containing unit with a gel-forming layer for forming a gel by water absorption; the drug-containing unit contains a brand-name (or original) pharmaceutical tablet (or a tablet having an equivalent formulation) and a physiologically acceptable effervescent agent. According to the process, the development period for applying the solid preparation of the present invention to a generic product can be shortened. Further, the solid preparation of the present invention can also be applied to all pharmaceutical preparations (or products).

In general, when a brand-name pharmaceutical tablet is newly covered with a covering layer, the elution property of the drug is significantly changed, so that the equivalence to the elution property of the drug of the brand-name tablet is not maintained. This causes a problem that Ministry of Health, Labour and Welfare does not approve the covered tablet as a branded generic product (what is called a generic product). In contrast, according to the solid preparation of the present invention, the equivalence to the brand-name tablet is easily obtainable.

For a solid preparation having a brand-name product (or a tablet having an equivalent formulation) as a drug-containing unit, the value of the $f_2$ function, which is represented by the following formula, described in Guideline for Bioequivalence Studies of Generic Products is, for example, preferably not less than 46 (not more than 100).

[Math. 1]

$$f_2 = 50 \log \left[ \frac{100}{\sqrt{1 + \dfrac{\sum\limits_{i=1}^{n} (Ti - Ri)^2}{n}}} \right]$$

wherein Ti represents an average dissolution rate of a drug from a control pharmaceutical preparation (the solid preparation of the present invention) at each point in time, Ri represents an average dissolution rate of a drug from a standard pharmaceutical preparation (brand-name pharmaceutical product) at each point in time, and n is the number of points in time at each of which these average dissolution rates are compared.

Further, as described above, the present invention includes a method for improving dissolution of a drug from a solid preparation which comprises a drug-containing unit (for example, a drug-containing unit containing a cationic or basic drug), a gel-forming layer forming a gel by water absorption (for example, a gel-forming layer containing an anionic or acidic polymer), and if necessary, an intermediate layer interposed between the drug-containing unit and the gel-forming layer, and the method comprises incorporating a physiologically acceptable effervescent agent in the drug-containing unit and/or the intermediate layer.

EXAMPLES

Hereinafter, the following examples are intended to describe this invention in further detail and should by no means be interpreted as defining the scope of the invention.

(Example 1)

(a) Step for producing anti-adhesive layer

A liquid coating composition A containing constituents of an anti-adhesive layer was prepared as follows.
To 380 parts by mass of purified water, 0.27 parts by mass of calcium chloride (Calcium chloride H, manufactured by Tomita Pharmaceutical Co., Ltd.) as a viscosity reducing agent was added and dissolved by stirring for 5 minutes. To this solution was slowly added 10.0 parts by mass of a polyacrylic acid (CARBOPOL 974P, manufactured byNoveon, viscosity of 0.2% bymass aqueous solution (20°C) : 12100 mPa·s) with stirring, and after the addition, the mixture was stirred for one hour. The mixture containing each component was heated to 80°C. To the mixture was slowly added 81.63 parts by mass of a hydroxypropylmethyl cellulose (TC-5E, manufactured by Shin-Etsu Chemical Co., Ltd., viscosity of 2% by mass aqueous solution (20°C) : 3 mPa·s) as an anti-adhesive agent with stirring. After the addition, the mixture was stirred for 15 minutes, and the temperature of the mixture was decreased to 30°C, and then the mixture was stirred for one hour. To the resulting mixture was added 8.1 parts by mass of glycerin (Japanese Pharmacopoeia, concentrated glycerin, manufactured by Asahi Denka Kogyo K.K.) as a water absorption promoter. After the addition, the mixture was stirred for 15 minutes to give a liquid coating composition A.
The liquid coating composition A was fully defoamed. A poly(ethylene terephthalate) film (SP-PET381031, manufactured by LINTEC Corporation), as a releasable substrate, had a releasably treated surface. The liquid coating composition A was spread-coated (spread-applied) on an untreated surface of the film using an applicator with an adjusted gap (the amount of the coating composition after drying: 30 g/m$^2$) and dried at 80°C for 10 minutes to form an anti-adhesive layer having a thickness of 28 $\mu$m after drying, and a laminate intermediate "a" (the anti-adhesive layer/the releasable substrate) was obtained.

(b) Step for producing gel-forming layer

A liquid coating composition B containing constituents of a gel-forming layer was prepared as follows.
To 700 parts by mass of purified water, 0.6 parts by mass of calcium chloride (Calcium chloride H, manufactured by Tomita Pharmaceutical Co., Ltd.) as a crosslinking agent was added and dissolved by stirring for 5 minutes. To this solution was slowly added 22.7 parts by mass of a polyacrylic acid (CARBOPOL 974P, manufactured byNoveon, viscosity of 0.2% bymass aqueous solution (20°C) : 12100 mPa·s) with stirring, and after the addition, the mixture was stirred for one hour. To the mixture was slowly added 68.6 parts by mass of a poly (vinyl alcohol) (GOHSENOL EG05T, manufactured by The Nippon Synthetic Chemical Industry Co., Ltd.) with stirring. After the addition, the mixture was stirred for 15 minutes, and then the mixture containing each component was heated to 80°C and stirred for one hour. Thereafter, the mixture containing each component was cooled to 30°C. To the mixture was added 8.1 parts by mass of glycerin (Japanese Pharmacopoeia, concentrated glycerin, manufactured by Asahi Denka Kogyo K.K.) as a water absorption promoter, and the resulting mixture was stirred for about 15 minutes to give a liquid coating composition B.

The liquid coating composition B was fullydefoamed. The liquid coating composition B was spread-coated (spread-applied) on the anti-adhesive layer formed in the step (a) using an applicator with an adjusted gap (the amount of the coating composition after drying: 10 g/m$^2$) and dried at 80°C for 6 minutes to give a laminate intermediate "b" (a laminate of the gel-forming layer/the anti-adhesive layer/the releasable substrate) having a gel-forming layer of 9 μm thickness after drying.

(c) Step for producing intermediate layer

A liquid coating composition C-1 containing constituents of an intermediate layer was prepared as follows.

To 190 parts by mass of water, 14.2 parts by mass of anhydrous calcium hydrogenphosphate as an electrolyte, 22.3 parts by mass of glycerin (Japanese Pharmacopoeia, concentrated glycerin, manufactured by Asahi Denka Kogyo K.K.) as a plasticizer were slowly added with stirring and dissolved. To the solution was slowly added 63.5 parts by mass of a polyvinylpyrrolidone (PVP K-90, manufactured by ISP Japan Ltd.) as a base material with stirring. After the addition, the mixture was stirred for 60 minutes to give a liquid coating composition C-1.

The liquid coating composition C-1 was fully defoamed. The liquid coating composition C-1 was spread-coated (spread-applied) on the gel-forming layer formed in the step (b) using an applicator with an adjusted gap (the amount of the coating composition after drying: 100 g/m$^2$) and dried at 80°C for 20 minutes to form an intermediate layer of 80 μm thickness after drying, and a laminate intermediate "c" (a laminate of the intermediate layer/the gel-forming layer/the anti-adhesive layer/the releasable substrate) was obtained.

(d) Step for forming drug-containing layer

Sodium hydrogencarbonate (22.5 parts by mass) as an effervescent agent, anhydrous calcium hydrogenphosphate (25 parts by mass) as an electrolyte, amlodipine besilate (2.9partsbymass) asabasicdrug, acarboxymethylcellulose (Japanese Pharmacopoeia Carmellose NS-300, manufactured by Gotoku Chemical Co., Ltd.) (10 parts by mass) as a disintegrant, and a crystalline cellulose (39.6 parts by mass) as a base material were fully mixed and dispersed in a mortar. The resulting powder was subjected to tablet compression by a tableting machine. On the other hand, the intermediate "c" (a first intermediate "c") was pressed from the intermediate layer side thereof to form a recessed area having a size capable of accommodating a tablet. The tablet (mass: 120 mg, drug content: 3.47 mg (containing 2.5 mg as amlodipine), tablet size: 8 mm diameter x 2 mm thickness) was accommodated in the recessed area and then covered with a second intermediate "c". The periphery of the intermediate layer of the first intermediate "c" and that of the second intermediate "c" were bonded to each other by thermal-adhering at 100°C under 1 kgf/cm$^2$ for 3 seconds. Insuchaprocess, a laminate having the releasable substrate/the anti-adhesive layer/the gel-forming layer/the intermediate layer/the tablet (drug-containing layer)/the intermediate layer/the gel-forming layer/the anti-adhesive layer/the releasable substrate in this order, which had the tablet included therein, was prepared. After the both releasable substrates were removed, a circular shape having a diameter of 15 mm was punched out of the laminate to produce a solid preparation (oral administration preparation) having a lamination structure. In the punching of the laminate, the thermally adhered region of the intermediate layers was punched to avoid exposure of the tablet.

(Example 2)

A solid preparation (oral administration preparation) was produced in the same manner as in Example 1 except that a commercially available amlodipine tablet (manufactured by Pfizer Inc., trade name "NORVASC", containing amlodipine besilate (containing 2.5 mg as amlodipine), tablet size: 6 mm diameter x 3 mm thickness) and 10 mg of sodium hydrogencarbonate as an effervescent agent were accommodated in the recessed area to form a drug-containing unit.

(Comparative Example 1)

Anhydrous calcium hydrogenphosphate (25 parts by mass) as an electrolyte, amlodipine besilate (2.9 parts by mass) asabasicdrug, acarboxymethylcellulose (Japanese Pharmacopoeia Carmellose NS-300, manufactured by Gotoku Chemical Co., Ltd.) (10 parts by mass) as a disintegrant, and a crystalline cellulose (62.1 parts by mass) as a base material were fully mixed and dispersed in a mortar. The resulting powder was subjected to tablet compression by a tableting machine, andatablet (mass:120 mg, drugcontent: 3.47 mg (containing 2.5 mg as amlodipine), tablet size: 8 mm diameter x 2 mm thickness) was obtained. In the same manner as in Example 1 except for using the resulting tablet as a drug-containing unit, a solid preparation (oral administration preparation) was produced.

(Example 3)

Anhydrous calcium hydrogenphosphate (25 parts by mass) as an electrolyte, amlodipine besilate (2.9 parts by mass) as a basic drug, and a crystalline cellulose (72.1 parts by mass) as a base material were fully mixed and dispersed in a mortar. The resulting powder was subjected to tablet compression by a tableting machine, and a tablet (mass: 120 mg, drug content: 3.47 mg (containing 2.5 mg as amlodipine), tablet size: 8 mm diameter x 2 mm thickness) was obtained. In the same manner as in Example 1 except for using the resulting tablet as a drug-containing unit and the following liquid coating composition C-2 as an intermediate layer, a solid preparation (oral administration preparation) was produced.

The liquid coating composition C-2 was prepared as follows. To 160 parts by mass of water, 10.0 parts by mass of sodium bicarbonate as an effervescent agent, 14.2 parts by mass of anhydrous calcium hydrogenphosphate as an electrolyte, 21.2 parts by mass of glycerin (Japanese Pharmacopoeia, concentrated glycerin, manufactured by Asahi Denka Kogyo K.K.) as a plasticizer were slowly added with stirring and dissolved. To the solution was slowly added 54.6 parts by mass of a polyvinylpyrrolidone (PVP K-90, manufactured by ISP Japan Ltd.) as a base material with stirring. After the addition, the mixture was stirred for 60 minutes to give a liquid coating composition C-2.

(Example 4)

A solid preparation (oral administration preparation) was produced in the same manner as in Example 3 except that a commercially available amlodipine tablet (manufactured by Pfizer Inc., trade name "NORVASC", containing amlodipine besilate (containing 2.5 mg as amlodipine), tablet size: 6 mm diameter x 3 mm thickness) was used as a drug-containing unit.

(Comparative Example 2)

In the same manner as in Example 3 except that the liquid coating composition C-1 was used as an intermediate layer, a solid preparation (oral administration preparation) was produced.

[Test method]

[Average dissolution rate of drug]

For each solid preparation (oral administration preparation) obtained in Examples 1 to 4 and Comparative Examples 1 to 2, the dissolution rate of the drug was measured by a method in accordance with Dissolution Test, the second method (Paddle Method) defined in Japanese Pharmacopoeia 15th edition. Incidentally, water was used as a dissolution medium. After stirring for each of 15 minutes, 30 minutes, 45 minutes, and 60 minutes at the number of revolutions of 50 rpm, each sample was collected and quantitatively analyzed by a high-speed liquid chromatography to determine the average dissolution rate of the drug on the basis of the amount of the drug supplied in a production of the solid preparation (oral administration preparation). The results are shown in Table 1.

[Equivalence to elution property of drug on the basis of comparison with standard pharmaceutical preparation]

For each solid preparation (oral administration preparation) obtained in Examples 1 to 4 and Comparative Examples 1 to 2, the equivalence to elution property of each preparation compared with the standard pharmaceutical preparation was determined. The acceptance/rejection criteria in this test were based on the $f_2$ function described in Guideline for Bioequivalence Studies of Generic Products. Specifically, a commercially available amlodipine tablet (manufactured by Pfizer Inc., trade name "NORVASC", containing amlodipine besilate (containing 2.5 mg as amlodipine), tablet size: 6 mm diameter x 3 mm thickness) was used as a standard pharmaceutical preparation, and when the $f_2$ value represented by the following formula was not less than 46, the preparation was determined to be equivalent (acceptance).

[Math. 2]

$$f_2 = 50 \log \left[ \frac{100}{\sqrt{1 + \dfrac{\displaystyle\sum_{i=1}^{n} (Ti - Ri)^2}{n}}} \right]$$

In the formula, Ti represents an average dissolution rate of a drug from each solid preparation of Examples 1 to 4 and Comparative Examples 1 to 2 at each sampling point in time, Ri represents an average dissolution rate of a drug from the standard pharmaceutical preparation at each sampling point in time, and n is the number of points in time at each of which these average dissolution rates are compared. In this test, the value of the $f_2$ function was calculated in the following conditions: n was 4, and the comparing point of time of the average dissolution rage of the drug was 15 minutes, 30 minutes, 45 minutes, and 60 minutes. The results are shown in Table 1.

[Table 1]

Table 1

| | Average dissolution rate of drug | | | | $f_2$ value | Equivalence to elution property of drug |
|---|---|---|---|---|---|---|
| | 15 min. | 30 min. | 45 min. | 60 min. | | |
| Example 1 | 78.0 | 81.4 | 81.5 | 82.8 | 50.7 | Acceptance |
| Example 2 | 80.0 | 81.0 | 81.8 | 82.0 | 49.2 | Acceptance |
| Comparative Example 1 | 19.0 | 47.0 | 57.8 | 66.7 | 28.1 | Rejection |
| Example 3 | 46.5 | 66.2 | 73.2 | 78.3 | 52.6 | Acceptance |
| Example 4 | 44.5 | 64.7 | 72.1 | 78.0 | 49.7 | Acceptance |
| Comparative Example 2 | 19.0 | 47.0 | 57.8 | 66.7 | 28.1 | Rejection |

As apparent from Table 1, the solid preparations of Examples 1 and 2, each of which contains the effervescent agent in the drug-containing unit, have an improved elution property of the drug compared with the solid preparation of Comparative Example 1, which is free from an effervescent agent. Moreover, the solid preparations of Examples 3 and 4, each of which contains the effervescent agent in the intermediate layer, have an improved elution property of the drug compared with the solid preparation of Comparative Example 2, which is free from an effervescent agent. In these examples, particularly the initial dissolution rate of the drug is markedly improved.

Moreover, the solid preparations of Examples 1 to 4 each have an $f_2$ value of not less than 46 and are determined to be acceptance on the basis of the equivalence to the elution property of the drug compared with the standard pharmaceutical preparation. Therefore, it is clear that the solid preparation of the present invention is suitable for a generic drug.

INDUSTRIAL APPLICABILITY

Since the solid preparation (oral administration preparation) of the present invention can improve the elution property of the drug, the bioavailability can be improved. Moreover, the solid preparation can easily be swallowed in the presence of a small quantity of water (such as saliva) due to a gel-forming layer thereof. Further, the anti-adhesive layer can effectively prevent the adhesion of the solid preparation to the inner wall of the oral cavity and can significantly improve the comfortability of taking the solid preparation.

DESCRIPTION OF REFERENCE NUMERALS

1 Solid preparation
2 Drug-containing unit
3 Intermediate layer
4 Gel-forming layer
5 Anti-adhesive layer

**Claims**

1.  A solid preparation comprising
    a drug-containing unit containing a drug and
    a gel-forming layer for covering the drug-containing unit and forming a gel by water absorption;
    wherein the drug-containing unit contains a physiologically acceptable effervescent agent.

2.  A solid preparation comprising
    a drug-containing unit containing a drug,
    a gel-forming layer for covering the drug-containing unit and forming a gel by water absorption, and
    an intermediate layer interposed between the drug-containing unit and the gel-forming layer;
    wherein at least one of the drug-containing unit and the intermediate layer contains a physiologically acceptable effervescent agent.

3.  A solid preparation according to claim 1 or 2, wherein the effervescent agent comprises a salt of at least one member selected from the group consisting of an alkali metal, an alkaline earth metal, and ammonia, wherein the salt is a carbonate, a bicarbonate, or a sesquicarbonate.

4.  A solid preparation according to any one of claims 1 to 3, wherein the ratio of the effervescent agent contained in the drug-containing unit is 0.1 to 160 parts by mass relative to 1 part by mass of the drug.

5.  A solidpreparation according to any one of claims 2 to 4, wherein the ratio of the effervescent agent contained in the intermediate layer is 0.01 to 50 parts bymass relative to 1 part by mass of the drug.

6.  A solidpreparation according to any one of claims 1 to 5, wherein the drug-containing unit contains a disintegrant.

7.  A solid preparation according to any one of claims 2 to 6, wherein the intermediate layer contains a disintegrant.

8.  A solid preparation according to claim 6 or 7, wherein the disintegrant comprises an acidic disintegrant.

9.  A solid preparation according to any one of claims 6 to 8, wherein the disintegrant comprises at least one member selected from the group consisting of a carboxymethyl cellulose, a carboxymethyl starch, and a cellulose acetate phthalate.

10. A solid preparation according to any one of claims 6 to 9, wherein, in the whole solid preparation, the ratio of the disintegrant is 10 to 80 parts by mass relative to 100 parts by mass of the effervescent agent.

11. A solid preparation according to any one of claims 1 to 10, wherein the drug-containing unit contains a cationic or basic drug, and the gel-forming layer contains an anionic or acidic polymer.

12. A solid preparation according to any one of claims 1 to 11, wherein the drug-containing unit contains a pharmaceutically acceptable electrolyte.

13. A solid preparation according to any one of claims 2 to 12, wherein the intermediate layer contains a pharmaceutically acceptable electrolyte.

14. A solid preparation according to any one of claims 1 to 13, which further comprises an anti-adhesive layer for covering the gel-forming layer directly or indirectly and dissolving in water to prevent adhesion of the solid preparation to an inner wall of an oral cavity.

15. A solid preparation according to any one of claims 1 to 14, which is a preparation in the form of a film.

**16.** A method for improving dissolution of a drug from a solid preparation which comprises a drug-containing unit containing the drug, and a gel-forming layer for covering the drug-containing unit and forming a gel by water absorption, the method comprising incorporating a physiologically acceptable effervescent agent into the drug-containing unit.

**17.** A method for improving dissolution of a drug from a solid preparation which comprises a drug-containing unit containing the drug, a gel-forming layer for covering the drug-containing unit and forming a gel by water absorption, and an intermediate layer interposed between the drug-containing unit and the gel-forming layer, the method comprising incorporating a physiologically acceptable effervescent agent into at least one of the drug-containing unit and the intermediate layer.

Fig. 1

## INTERNATIONAL SEARCH REPORT

| | International application No. |
|---|---|
| | PCT/JP2011/056055 |

**A. CLASSIFICATION OF SUBJECT MATTER**

*A61K9/46*(2006.01)i, *A61K9/28*(2006.01)i, *A61K9/70*(2006.01)i, *A61K47/02*(2006.01)i, *A61K47/04*(2006.01)i, *A61K47/30*(2006.01)i, *A61K47/38*(2006.01)i

According to International Patent Classification (IPC) or to both national classification and IPC

**B. FIELDS SEARCHED**

Minimum documentation searched (classification system followed by classification symbols)
A61K9/46, A61K9/28, A61K9/70, A61K47/02, A61K47/04, A61K47/30, A61K47/38

Documentation searched other than minimum documentation to the extent that such documents are included in the fields searched
Jitsuyo Shinan Koho          1922-1996   Jitsuyo Shinan Toroku Koho   1996-2011
Kokai Jitsuyo Shinan Koho    1971-2011   Toroku Jitsuyo Shinan Koho   1994-2011

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2005/097080 A1 (Lintec Corp.), 20 October 2005 (20.10.2005), paragraphs [0015] to [0057]; examples; fig. 1 to 4 <br> & JP 2005-289868 A     & US 2007/0141152 A1 <br> & EP 1757273 A1         & KR 10-2006-0135052 A <br> & CN 1956708 A | 1-17 |
| Y | JP 2005-519865 A (Ranbaxy Laboratories Ltd.), 07 July 2005 (07.07.2005), claims 1, 11 to 13 <br> & EP 1432410 A          & WO 2003/026610 A2 <br> & CN 1578657 A | 1-17 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26 July, 2011 (26.07.11) | 02 August, 2011 (02.08.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2011/056055 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 9-221416 A  (JAGOTEC AG.),<br>26 August 1997 (26.08.1997),<br>paragraphs [0019], [0021]<br>& US 6294200 B1          & EP 788790 A2 | 1-17 |
| Y | JP 2002-543109 A  (Cima Labs Inc.),<br>17 December 2002 (17.12.2002),<br>paragraph [0004]; fig. 2 to 4<br>& US 6350470 B1          & US 6509036 B2<br>& US 2002/0076439 A1     & US 6391335 B1<br>& US 6576250 B1          & US 2003/0118645 A1<br>& US 2003/0133976 A1     & US 2005/0037072 A1<br>& US 6139874 A           & EP 1091732 A<br>& WO 2000/066089 A1 | 1-17 |
| A | JP 62-207208 A  (Teijin Ltd.),<br>11 September 1987 (11.09.1987),<br>(Family: none) | 1-17 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 63264518 A
- JP 7277959 A
- JP 4372347 B
- WO 2002087622 A
- WO 2005097080 A
- JP 2008037794 A

**Non-patent literature cited in the description**

- Japanese Pharmacopoeia